# EUROPEAN PATENT SPECIFICATION

(11) **EP 1 219 599 B1**
(45) Date of publication and mention of the grant of the patent: **31.03.2010**
(21) Application number: 00955023.7
(22) Date of filing: 25.08.2000
(51) Int. Cl.: C07C 401/00, A61K 31/592, A61P 3/02, A61P 3/14, A61P 35/00, A61P 37/02

(54) **VITAMIN D DERIVATIVES HAVING SUBSTITUENTS AT THE 2 ALPHA-POSITION**
VITAMIN-D-DERIVATE MIT SUBSTITUENTEN AN DER 2-ALPHA-POSITION
DERIVES DE VITAMINE D AYANT DES SUBSTITUANTS A LA POSITION 2 ALPHA

(30) Priority: 27.08.1999 JP 24165099
(43) Date of publication of application: 03.07.2002
(73) Proprietor: Takayama, Hiroaki, Tokyo 151-0072 (JP)
(72) Inventor: TAKAYAMA, Hiroaki, Shibuya-ku, Tokyo 151-0072 (JP); FUJISHIMA, Toshie, Hachioji-shi, Tokyo 193-0834 (JP); SUHARA, Yoshitomo, Yokohama-shi, Kanagawa 226-0025 (JP); NIHEI, Ken-Ichi, Tsukui-gun, Kanagawa 220-0105 (JP); KONNO, Katsuhiro Center of Study of Social Insects, 1515 Rio Claro, SP 13506-900 (BR)
(74) Representative: Vossius & Partner
(86) International application number: PCT/JP2000/005743
(87) International publication number: WO 2001/016099

(56) References cited:
- WO-A-00/66548
- WO-A1-96/01811
- JP-A- 6 041 059
- US-A- 5 877 168
- GARY H. POSNER ET.AL.: "Stereocontrolled Total Synthesis of Calcitriol Derivatives: 1,25-Dihydroxy-2-(4'hydroxybutyl)vitamin D3 Analogs of an Osteoporosis Drug" J. ORG. CHEM., vol. 59, - 17 June 1994 (1994-06-17) pages 7855-7861, XP002205771
- ONO YOSHIYUKI ET AL.: 'Synthetic studies of vitamin D analogs. XXIV. Synthesis of active vitamin D3 analogs substituted at the 2beta-position and their preventive effects on bone mineral loss in ovariectomized rats' CHEM. PHARM. BULL. vol. 45, no. 10, 1997, pages 1626 - 1630, XP002937868
- SUHARA YOSHITOMO ET AL.: 'Syntheses and biological evaluation of novel 2alpha-substituted 1alpha,25-dihydroxyvitamin D3 analogues' BIOORG. MED. CHEM. LETT. vol. 10, no. 10, 2000, pages 1129 - 1132, XP002937869

## Description

The present invention relates to novel vitamin D derivatives, more particularly, relates to vitamin D derivatives having a substituent at the 2α-position.

### BACKGROUND ART

Active vitamins D₃ including 1α,25-dihydroxyvitamin D₃ are known to exhibit a variety of physiological activities such as calcium metabolism regulatory activities, growth inhibitory and differentiation toward tumor cells, and immunoregulatory activities. However, some active vitamins D₃ disadvantageously may cause hypercalcemia during long-term and continuous administration, and are thus not suitable for use as antitumor agents, antirheumatic agents, or the like. Thus, a number of synthetic studies have been conducted for the purpose of obtaining vitamin D derivatives which are superior in certain activities.

The studies conducted by the inventors of the present invention revealed that 2α-methylderivatives of active vitamin D₃, i.e., 1α,25-dihydroxyvitamin D₃, increases binding property to vitamin D receptor (VDR) (K.Konno, et al., Bioorg. Med. Chem. Lett., 1998, 8, 151). Furthermore, a combination of the 2α-methylation and epimerization at the 20-position on the side chain has been reported to additionally enhance the binding property to VDR (T.Fujishima et al., Bioorg. Med. Chem. Lett., 1998, 8, 2145).

WO-A1-9601811 discloses Vitamin D3 analogues which include a 2-substituted alcohol or fluoride.

GARY H. POSNER ET. AL describes the stereocontrolled total synthesis of Calcitriol Derivates: 1,25-Dihydroxy-2-(4'hydroxybutyl)vitamin D3 Analogs of an Osteoporosis Drug in J. Org. Chem., vol. 59, 1994, p. 7855-7861.

ONO YOSHIYUKI ET. AL pertains to the synthesis of active vitamin D3 analogs substituted at the 2beta-position and their preventive effects on bone mineral loss in ovariectomized rats in Chem. Pharm. Bull., vol. 45, no. 10, 1997, p. 1626 - 1630.

### DISCLOSURE OF THE INVENTION

An object of the present invention is to synthesize and provide a novel vitamin D₃ derivative, in which the 2-position is substituted, other than with a methyl group, the hydroxy groups at 1- and 3-positions are α- and β-oriented, respectively, and the groups at the 20-position are epimerized.

Another object of the present invention is to evaluate bioavailability of the synthesized vitamin D₃ derivative.

As a result of careful studies to achieve the above objects, the inventors of the present invention have succeeded in synthesizing vitamin D derivatives having a variety of configurations by synthesizing CD-ring compounds each having a desired side chain portion and then coupling it with A-ring compounds each having a desired group at the 2α-position, thereby completing the present invention.

According to an aspect of the present invention, a vitamin D derivative represented by Formula (I) is provided: 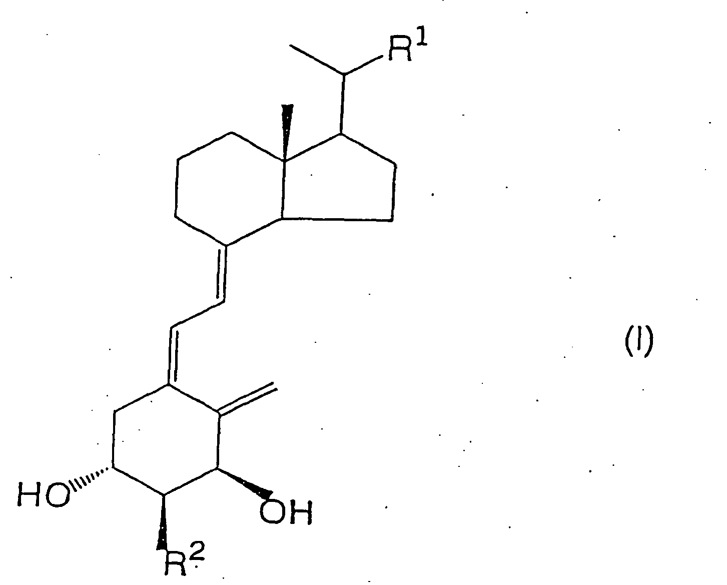 wherein
R¹ represents a saturated aliphatic C₁₋₁₅hydrocarbon group optionally substituted with 1 to 3 hydroxy or protected hydroxy groups; and
R² represents a saturated aliphatic C₁₋₁₀hydrocarbon group optionally substituted with one or more substituents, which may be the same or different and which are selected from the group consisting of a cyano group, a lower alkoxy group, an amino group and an acylamino group, provided that when R² represents a saturated aliphatic C₁hydrocarbon group, R² is substituted with at least one of said substituents.

Among such vitamin D derivatives, those represented by the following Formula (II) or 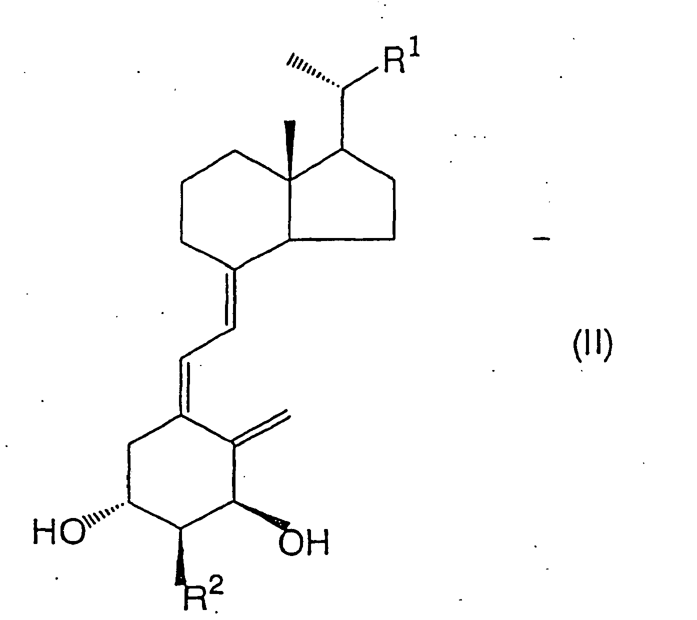 (III) are preferred.

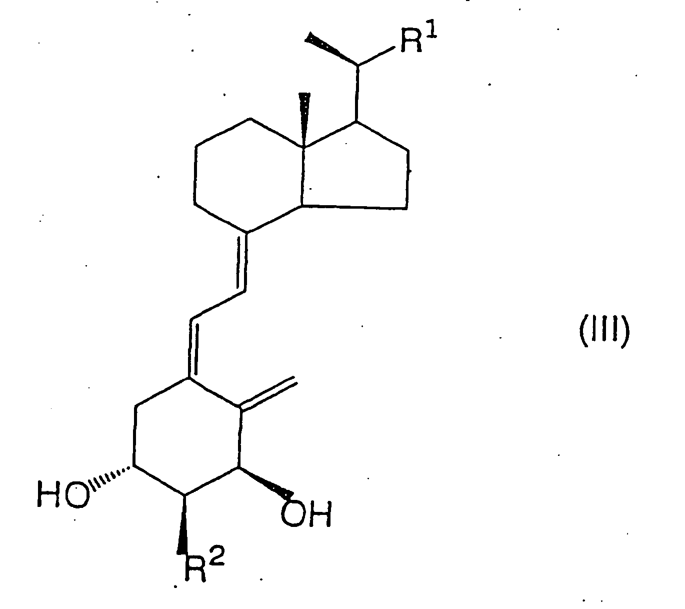

In Formulae (I), (II) and (III), R² is preferably a ethyl, propyl, butyl, pentyl or hexyl group.

In Formulae (I), (II) and (III), R¹ is preferably a saturated aliphatic C₁₋₁₅hydrocarbon group substituted with one hydroxy or protected hydroxy group, more preferably R¹ is a 4-hydroxy-4-methylpentyl group.

Particularly preferred compounds of the present invention are those selected from the group consisting of (5Z,7E)-(1S,2S,3R,20R)-9,10-seco-5,7,10(19)-cholestatriene-2-ethyl-1,3,25-triol, (5Z,7E)-(1S,2S,3R,20R)-9,10-seco-5,7,10(19)-cholestatriene-2-propyl-1,3,25-triol, (5Z,7E)-(1S,2S,3R,20R)-9,10-seco-5,7,10(19)-cholestatriene-2-butyl-1,3,25-triol, (5Z,7E)-(1S,2S,3R,20R)-9,10-seco-5,7,10(19)-cholestatriene-2-pentyl-1,3,25-triol and (5Z,7E)-(1S,2S,3R,20R)-9,10-seco-5,7,10(19)-cholestatriene-2-hexyl-1,3,25-triol.

According to the present invention, a pharmaceutical composition comprising the above mentioned vitamin D derivative as an active ingredient is provided. Examples of such medicaments include therapeutic agents for diseases accompanied with abnormal calcium metabolism, an antitumor agent, an immunomodulator, and the like.

According to the present invention, use of the above mentioned vitamin D derivative for a medicament is provided. Examples of such medicaments include therapeutic agents for diseases accompanied with abnormal calcium metabolism, an antitumor agent, an immunomodulator, and the like.

### PREFERRED MODE FOR CARRYING OUT THE INVENTION

Detailed modes and methods with respect to vitamin D derivatives represented by Formula (I) and pharmaceutical compositions including the vitamin D derivatives in accordance with the present invention are described in further detail below.

The contents of the specification of Japanese Patent Application No.11-241650, the application on the basis of which the present application claims priority are to be incorporated in their entirety by reference.

In Formula (I), R¹ represents a saturated aliphatic C₁₋₁₅hydrocarbon group which may be substituted with 1 to 3 hydroxy groups or protected hydroxy groups.

In the present application, a saturated aliphatic hydrocarbon group generally means a straight or branched alkyl group. Specific examples thereof include methyl, ethyl, n-propyl, i-propyl, n-butyl, s-butyl, i-butyl and tert-butyl groups, and further include pentyl, hexyl, heptyl, octyl, nonyl and decanyl groups. Among them, 3-methylbutyl, 3-ethylpentyl, 4-methylpentyl, 3-(n-propyl)hexyl, 4-ethylhexyl, 5-methylhexyl, 6-methylheptyl, 5-ethylheptyl and 4-(n-propyl)hepty groups are preferred.

R¹ is preferably a 5-methylhexyl, 4-ethylhexyl or 4-methylpentyl group.

The saturated aliphatic hydrocarbon groups optionally substituted with hydroxy groups means that any hydrogen atoms in the above mentioned saturated hydrocarbon groups may be substituted with one or more hydroxy groups.

In the definition of R¹, the number of hydroxy substituents is 0, 1, 2 or 3, preferably 1 or 2, and more preferably 1. Specific examples of the saturated aliphatic hydrocarbon groups substituted with at least one hydroxy group include 2-hydroxy-2-methylpropyl; 3-hydroxy-2-methylpropyl, 2,3-dihydroxy-2-methylpropyl, 2-ethyl-2-hydroxybutyl, 2-ethyl-3-hydroxybutyl, 2-ethyl-2,3-dihydroxybutyl, 2-hydroxy-2-(n-propyl)pentyl, 3-hydroxy-2-(n-propyl)pentyl, 2,3-dihydroxy-2-(n-propyl)pentyl, 2-hydroxy-3-methylbutyl, 3-hydroxy-3-methylbutyl, 4-hydroxy-3-methylbutyl, 2,3-dihydroxy-3-methylbutyl, 2,4-dihydroxy-3-methylbutyl, 3,4-dihydroxy-3-methylbutyl, 3-ethyl-2-hydroxypentyl, 3-ethyl-3-hydroxypentyl, 3-ethyl-4-hydroxypentyl, 3-ethyl-2,3-dihydroxypentyl, 3-ethyl-2,4-dihydroxypentyl, 3-ethyl-3,4-dihydroxypentyl, 2-hydroxy-3-(n-propyl)hexyl, 3-hydroxy-3-(n-propyl)hexyl, 4-hydroxy-3-(n-propyl)hexyl, 2,3-dihydroxy-3-(n-propyl)hexyl, 2,4-dihydroxy-3-(n-propyl)hexyl, 3,4-dihydroxy-3-(n-propyl)hexyl, 3-hydroxy-4-methylpentyl, 4-hydroxy-4-methylpentyl, 5-hydroxy-4-methylpentyl, 3,4-dihydroxy-4-methylpentyl, 3,5-dihydroxy-4-methylpentyl, 4,5-dihydroxy-4-methylpentyl, 4-ethyl-3-hydroxyhexyl, 4-ethyl-4-hydroxyhexyl, 4-ethyl-5-hydroxyhexyl, 4-ethyl-3,4-dihydroxyhexyl, 4-ethyl-3,5-dihydroxyhexyl, 4-ethyl-4,5-dihydroxyhexyl, 3-hydroxy-4-(n-propyl)heptyl, 4-hydroxy-4-(n-propyl)heptyl, 5-hydroxy-4-(n-propyl)heptyl, 3,4-dihydroxy-4-(n-propyl)heptyl, 3,5-dihydroxy-4-(n-propyl)heptyl, 4,5-dihydroxy-4-(n-propyl)heptyl, 4-hydroxy-5-methylhexyl, 5-hydroxy-5-methylhexyl, 6-hydroxy-5-methylhexyl, 4,5-dihydroxy-5-methylhexyl, 4,6-dihydroxy-5-methylhexyl, 5,6-dihydroxy-5-methylhexyl, 5-ethyl-4-hydroxyheptyl, 5-ethyl-5-hydroxyheptyl, 5-ethyl-6-hydroxyheptyl, 5-ethyl-4,5-dihydroxyheptyl, 5-ethyl-4,6-dihydroxyheptyl, 5-ethyl-5,6-dihydroxyheptyl, 4-hydroxy-5-(n-propyl)octyl, 5-hydroxy-5-(n-propyl)octyl, 6-hydroxy-5-(n-propyl)octyl, 4,5-dihydroxy-5-(n-propyl)octyl, 4,6-dihydroxy-5-(n-propyl)octyl, 5,6-dihydroxy-5-(n-propyl)octyl, 5-hydroxy-6-methylheptyl, 6-hydroxy-6-methylheptyl, 7-hydroxy-6-methylheptyl, 5,6-dihydroxy-6-methylheptyl, 5,7-dihydroxy-6-methylheptyl, 6,7-dihydroxy-6-methylheptyl, 6-ethyl-5-hydroxyoctyl, 6-ethyl-6-hydroxyoctyl, 6-ethyl-7-hydroxyoctyl, 6-ethyl-5,6-dihydroxyoctyl, 6-ethyl-5,7-dihydroxyoctyl, 6-ethyl-6,7-dihydroxyoctyl, 5-hydroxy-6-(n-propyl)nonyl, 6-hydroxy-6-(n-propyl)nonyl, 7-hydroxy-6-(n-propyl)nonyl, 5,6-dihydroxy-6-(n-propyl)nonyl, 5,7-dihydroxy-6-(n-propyl)nonyl and 6,7-dihydroxy-6-(n-propyl) groups. Among them, 4-hydroxy-4-methylpentyl, 4-ethyl-4-hydroxyhexyl, 5-hydroxy-5-methylhexyl and 5-ethyl-5-hydroxyheptyl groups are more preferred and a 4-hydroxy-4-methylpentyl group is further more preferred.

Examples of protecting groups for the hydroxy groups in R¹ of Formulae (I), (II) and (III) include acyl groups, substituted silyl groups and substituted alkyl groups, preferably acyl groups and substituted silyl groups.

Acyl groups mean substituted carbonyl groups; examples of the substituents of the carbonyl groups include a hydrogen atom, optionally substituted lower alkyl groups, optionally substituted aryl groups, optionally substituted lower alkyloxy groups, optionally substituted aryloxy groups, optionally substituted aralkyloxy groups and the like. Preferred examples of the acyl groups include a formyl group, lower alkylcarbonyl groups, optionally substituted phenylcarbonyl groups, lower alkyloxycarbonyl groups, optionally substituted phenylalkyloxycarbonyl groups and the like, more preferred examples include formyl, acetyl, propionyl, butyryl, pivaloyl, benzoyl, methoxycarbonyl, ethoxycarbonyl, tert-butoxycarbonyl and benzyloxycarbonyl groups.

Substituted silyl groups mean silyl groups substituted with lower alkyl groups which may have one or more substituents, optionally substituted aryl groups and the like. Preferably, substituted silyl groups mean trisubstituted silyl groups. Preferred examples of the substituted silyl groups include trimethylsilyl, triethylsilyl, triisopropylsilyl, tert-butyldiphenylsilyl and tert-butyldimethylsilyl groups.

Substituted alkyl groups mean those alkyl groups which are substituted with one or more substituents. Preferred examples of the substituents include optionally substituted alkyloxy groups and optionally substituted aryl groups, with optionally substituted alkyloxy groups being particularly preferred. Examples of the alkyl groups substituted with an optionally substituted alkyloxy group (such as an alkyloxy group) include, for example, methoxymethyl, 2-methoxyethoxymethyl and tetrahydropyran-2-yl groups. Examples of the substituents include halogen atoms and cyano, nitro, amino, hydroxy, alkyl, alkyloxy, acyloxy and sulfonyl groups and the like.

In Formulae (I), (II) and (III), R² represents a saturated aliphatic hydrocarbon group which may be substituted with one or more of substituents selected from the group consisting of cyano, lower alkoxy, amino and acylamino groups.

Examples of the saturated aliphatic hydrocarbon groups include the above described straight or branched alkyl groups, preferably having 1-10 carbon atoms, more preferably 1-6 carbon atoms, and particularly preferably 3-5 carbon atoms.

The lower alkoxy groups mean those having 1-6 carbon atoms. The amino group may have protecting groups.

Similar to the above, preferred examples of the acyl groups of the acylamino group include a formyl group, lower alkylcarbonyl groups, optionally substituted phenylcarbonyl groups, lower alkyloxycarbonyl groups, optionally substituted phenylalkyloxycarbonyl groups and the like, more preferably formyl, acetyl, propionyl, butyryl, pivaloyl, benzoyl, methoxycarbonyl, ethoxycarbonyl, tert-butoxycarbonyl and benzyloxycarbonyl groups.

The number of substituents is 0, 1, 2 or 3, preferably 1 or 2, and more preferably 1.

It is particularly preferred that R² is a ethyl, propyl, butyl, pentyl or hexyl group.

The stereochemstry of the hydroxy groups at the 1-and 3-positions and the methyl group at the 2-position in compounds of Formula (I) of the present invention can occur in α or β conformation, and the respective compounds are all included within the scope of the present invention.

Examples of the compounds of Formula (I) of the present invention include 1α,25-dihydroxy-2α-ethylvitamin D₃, 1α,25-dihydroxy-2α-propylvitamin D₃, 1α,25-dihydroxy-2α-butylvitamin D₃, 1α,25-dihydroxy-2α-pentylvitamin D₃ and 1α,25-dihydroxy-2α-hexylvitamin D₃. Particularly preferred examples include (5Z,7E)-(1S,2S,3R,20R)-9,10-seco-5,7,10(19)-cholestatriene-2-ethyl-1,3,25-triol, (5Z,7E)-(1S,2S,3R,20R)-9,10-seco-5,7,10(19)-cholestatriene-2-propyl-1,3,25-triol, (5Z,7E)-(1S,2S,3R,20R)-9,10-seco-5,7,10(19)-cholestatriene-2-butyl-1,3,25-triol, (5Z,7E)-(1S,2S,3R,20R)-9,10-seco-5,7,10(19)-cholestatriene-2-pentyl-1,3,25-triol and (5Z,7E)-(1S,2S,3R,20R)-9,10-seco-5,7,10(19)-cholestatriene-2-hexyl-1,3,25-triol.

Although there is no limitation with respect to methods for synthesizing the compounds represented by Formula (I) of the present invention, for example, A-ring and CD-ring parts of a vitamin D derivative are separately synthesized and then subjected to coupling, as described in the following Examples.

CD-ring part compounds of the vitamin D derivatives are known. A desired CD-ring compound can be obtained by appropriately modifying a side chain of a known CD-ring compound or can be obtained from a known CD-ring compound having a corresponding side chain.

These known vitamin D derivatives are disclosed in Japanese Patent Publication (Kokai) Nos. 61-267550, 6-72994 and 6-256300, Japanese Patent Publication (Kohyo) Nos. 4-503669 and 4-504573, and Japanese Patent Publication (Kokai) No. 10-182597, WO 94/14766, WO 95/27697, and the like. After protecting the hydroxy group of the above mentioned vitamin D derivative with a protecting group, the resultant compound is subjected to ozonolysis, and then, to NaBH₄ reduction, giving an alcohol having a hydroxy group at the 8-position. Oxidization using an appropriate oxidant gives a ketone having an oxo group at the 8-position. A CD-ring compound having a desired side chain can be obtained by converting the oxo group at the 8-position with a bromomethylene group.

A-ring compounds having a substituent at the 2β-position are known and are described by K. Konno et al. (Bioorg. Med. Chem. Lett., 8(1998) P.151-156) and T. Fujishima et al. (Bioorg. Med. Chem. Lett., 8(1998), P.2145-2148).

A-ring compounds having a substituent at the 2α-position can be synthesized from a starting material, such as 1,2-O-isopropylidene-α-D-(R)-xylofuranose, according to the process described in the following Examples, however there is no limitation with respect to a method for synthesizing the compounds. Compounds other than those described in the following Examples can also be synthesized in the same manner by using corresponding starting materials.

Coupling of an A-ring compound with a CD-ring compound can be carried out using a known method. An A-ring compound, which is obtainable by the above method and which has a triple bond at one terminal and a double bond at the other terminal, is reacted with a CD-ring compound, which has a bromomethylene group at the coupling site for the A-ring compound, in the presence of a palladium catalyst such as Pd₂(dba)₃ and triphenylphosphine (PPh₃) in an appropriate solvent.

After the coupling reaction, the resulting product is purified in a usual manner such as thin layer chromatography and subjected to removal of the hydroxy protecting group, to give a desired vitamin D derivative having a substituent at the 2α-position.

The compounds of the present invention are preferably formulated into appropriate dosage forms with pharmaceutically acceptable carriers, excipients, disintegrants, lubricants, binders, flavors, colorants and the like; examples of the dosage forms include tablets, granules, fine granules, capsules, powders, injections, solutions, suspensions, emulsions, percutaneous administration formulations, suppositories and the like.

There is no restriction on routes of administration for the compounds of the present invention; the compounds may be administered orally or parenterally (intravenously, intramuscularly, intraperitoneally, percutaneously and the like).

Dosage of compounds of the present invention can be appropriately chosen depending on target disease, conditions, body type, constitution, age and sex of the patient, administration route, dosage form and other factors. Typically, the lower limit for an adult ranges from 0.001 µg to 0.1 µg and preferably around 0.01 µg daily, and the upper limit for an adult ranges from 100µg to 10000 µg and preferably from 200 µg to 1000 µg daily, which may be administered at a time or in divided portions twice or three times a day.

### EXAMPLES

The present invention will be described specifically by way of the following Examples, which in no way limit the invention.

### (Example 1): Synthesis of an A-ring compound used for the synthesis of a vitamin D derivative having a substituent at the 2α-position.

Example 1 was conducted according to the following reaction scheme:

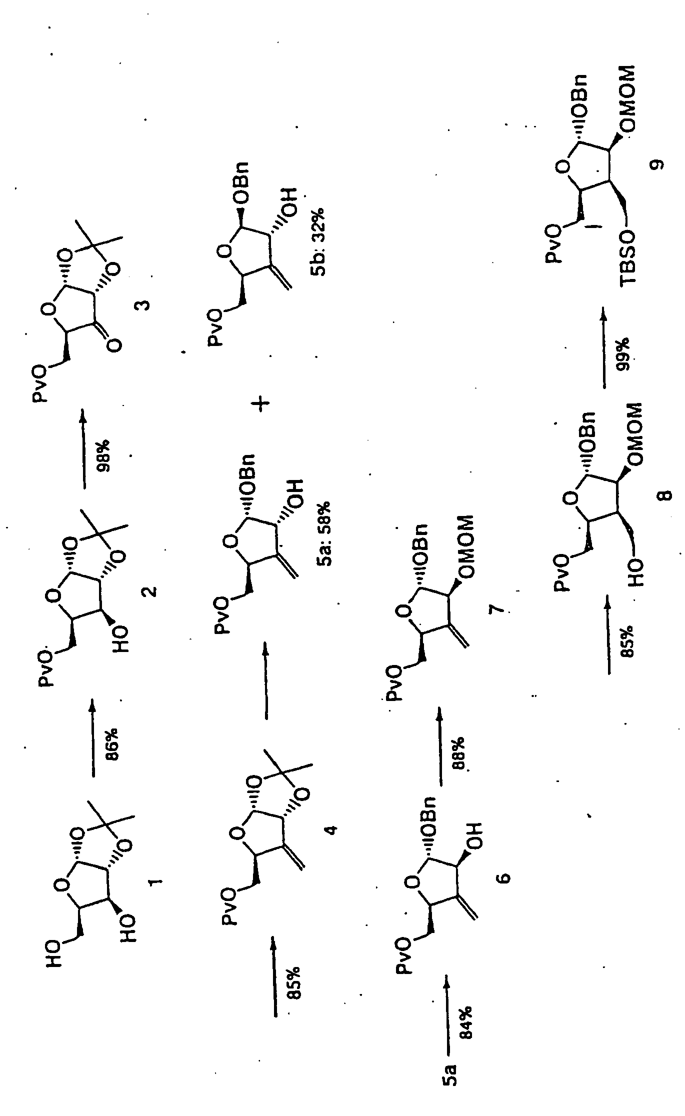

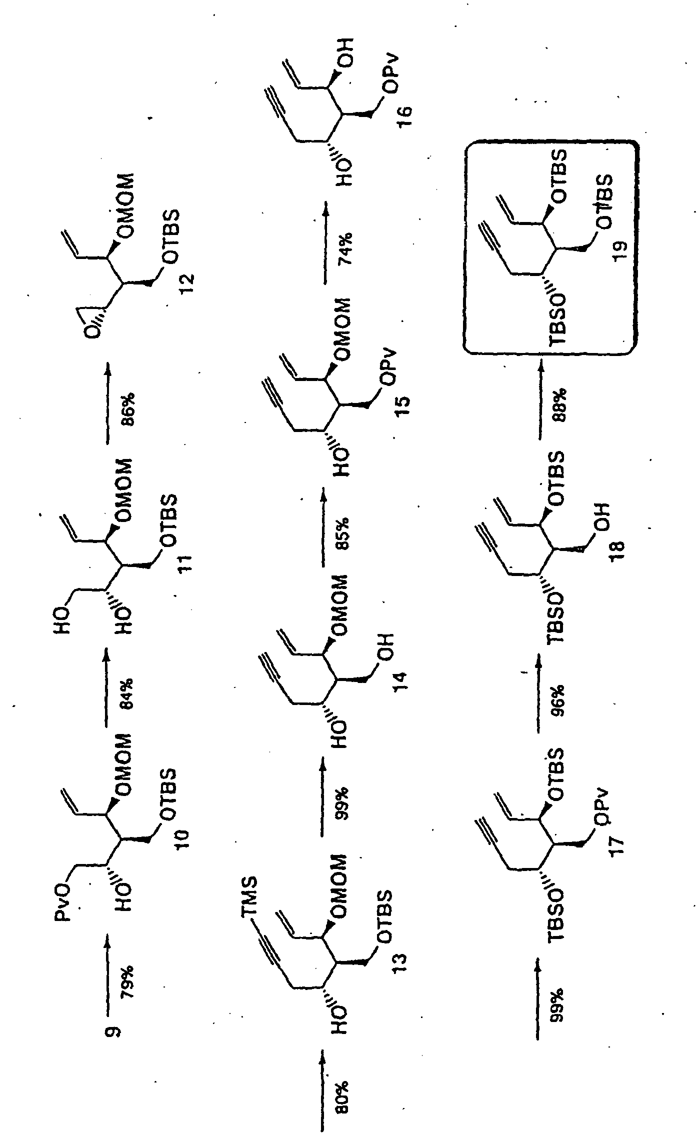

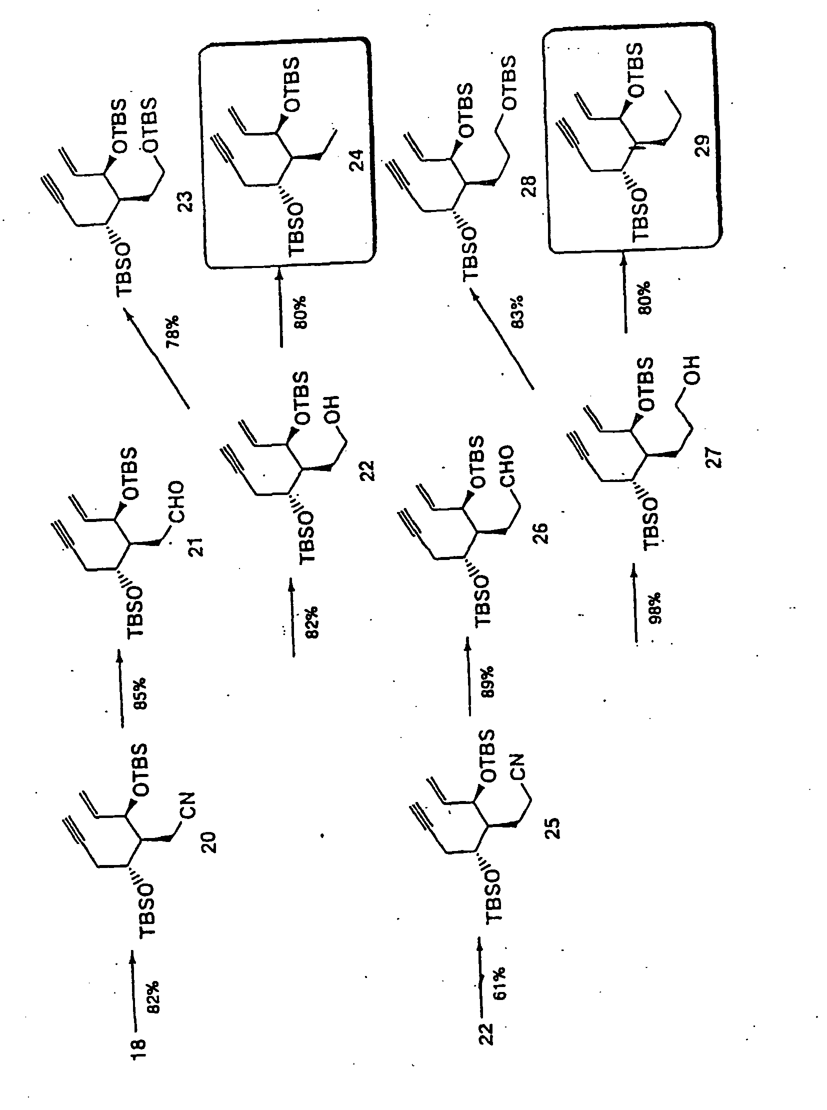

### (1) Synthesis of 5-O-pivalbyl-1,2-O-isopropylidene-α-D-xylofuranose (Compound 2)

1,2-O-isopropylidene-α-D-xylofuranose (Compound 1, 15.0 g, 78.9 mmol) was dissolved in pyridine (70 mL), cooled to 0°C under argon atmosphere, and trimethylacetyl chloride (9.9 g, 82.2 mmol) was added dropwise over 2 hours. The reaction mixture was stirred for 10 hours at that temperature, then MeOH (5 ml) was added and the mixture was concentrated. The residue was dissolved in diethyl ether (500 mL) and washed with water (100 mL), saturated aqueous copper sulfate solution (100 mL), water (100 mL) and saturated brine (100 mL). The aqueous layer was extracted with 100 mL of diethyl ether three times. The ether layers were combined with the previously obtained diethyl ether layer. The ether layer thus obtained was dried over magnesium sulfate, filtered and concentrated. The residue was purified by silica gel chromatography (ethyl acetate/hexane 1:4) to give Compound 2 (18.7 g, 86%) as a colorless oil. [α]²⁰ _{D} 2.69 (c 1.00, CHCl₃) ; ¹H NMR (400 MHz, CDCl₃ δ 1.22 (s, 9H), 1.32 (s, 3H), 1.51 (s, 3H), 4.10 (d, 1H, J = 2.8 Hz), 3.78 (bs, 1H), 4.17 (dd, 1H, J= 5.6, 11.2 Hz), 4.25 (ddd, 1H, J= 2.8, 5.6, 7.2 Hz), 4.50 (dd, 1H, J= 7.2, 11.2 Hz), 4.56 (d, 1H, J= 3.6 Hz), 5.93 (d, 1H, J= 3.6 Hz); HREIMS C₁₂H₁₉O₆ (M⁺-CH₃) calcd. 259.1182, found 259.1182.

### (2) Synthesis of 5-O-pivaloyl-1,2-O-isopropylidene-a-D-erythro-3-pentofuranose (Compound 3)

Compound 2 (10.5 g, 38.3 mmol) was dissolved in dichloromethane (800 mL), zeolite (75 g) and PCC (pyridinium chlorochromate) (36.5 g, 169.4 mmol) were added at room temperature and the mixture was stirred for 3 hours. The reaction mixture was diluted with hexane (800 mL), filtered and concentrated. The residue was purified by silica gel chromatography (ethyl acetate/hexane 2:3) to give ketone Compound 3 (10.2 g, 98%) as a colorless oil. [α]²⁰ _{D} 4.19 (c 1.13, CHCl₃) ; ¹H NMR (400 MHz, CDCl₃) δ 1.18 (s, 9H), 1.44 (s, 3H), 1.48 (s, 3H), 4.23 (d, 1H, J = 3.2, 11.6 Hz), 4.37 (dd, 1H, J= 1.2, 3.2 Hz), 4.39 (dd, 1H, J= 3.2, 11.6 Hz), 4.57 (dt, 1H, J= 1.2, 3.2 Hz), 6.10 (d, 1H, J= 4.4 Hz); HREIMS C₁₃H₂₀O₆ (M⁺) calcd. 276.1260, found 276.1262.

### (3) Synthesis of 5-O-pivaloyl-1,2-O-isopropylidene-3-deoxy-3-C-methylene-α-D-pentofuranose (Compound 4)

Methyltriphenylphosphonium bromide (7.7 g, 21.6 mmol) was mixed with THF (100 mL), and 1.0M NaHMDS (sodium bis(trimethylsilyl)amide) in THF (18.0 mL, 18.0 mmol) was added dropwise at room temperature. The yellow reaction suspension was stirred for 1.5 hours at room temperature, cooled to -78°C under argon atmosphere, and Compound 3 (4.3 g, 15.8 mmol) was slowly added. The reaction mixture was stirred at that temperature for 30 min., then warmed to room temperature and stirred for further 1 hour. The reaction mixture was cooled to 0°C, then MeOH (10 mL) was added. The mixture was diluted with diethyl ether (200 mL), and washed with a saturated aqueous ammonium chloride solution (100 mL) and with saturated brine (100 mL), each three times. The aqueous layer was extracted with 100 mL of diethyl ether three times and combined with the previously obtained ether layer. The ether layer was dried over magnesium sulfate, filtered and concentrated. The residue was purified by flash column chromatography (ethyl acetate/hexane 1:9) to give olefin Compound 4 (3.6 g, 85%) as a colorless oil. [α]²⁰ _{D} 13.45 (c 1.31, CHCl₃); ¹H NMR (400 MHz, CDCl₃) δ 1.20 (s, 9H), 1.39 (s, 3H), 1.52 (s, 3H), 4.19 (dd, 1H, J = 4.8, 12.0 Hz), 4.23 (dd, 1H, J= 3.6, 12.0 Hz), 4.91 (dd, 1H, J= 1.2, 4.0 Hz), 4.94 (td, 1H, J= 2.6, 3.4, 4.8 Hz), 5.22 (t, 1H, J= 1.2 Hz), 5.48 (dd, 1H, J= 1.2, 2.4 Hz), 5.87 (d, 1H, J= 4.0 Hz); HREIMS C₁₃H₁₉O₅ (M⁺-Me) calcd. 255.1235, found 255.1232.

### (4) Synthesis of benzyl 5-O-pivaloyl-3-deoxy-3-C-methylene-α-D-xylo-pentofuranose (Compound 5a) and benzyl 5-O-pivaloyl-3-deoxy-3-C-methylene-β-D-xylo-pentofuranose (Compound 5b)

Compound 4 (3.2 g, 11.9 mmol) and benzyl alcohol (8.0 g, 74.1 mmol) were dissolved in toluene (22 mL), cooled to 0°C, and 4.0M hydrogen chloride-dioxane solution (10 mL, 40.0 mmol) was added. The reaction mixture was warmed to room temperature, stirred for 16 hours and diluted with a diethyl ether (200 mL). The diluted solution was cooled to 0°C and neutralized with saturated aqueous sodium bicarbonate solution (100 mL). The organic layer was washed with water (50 mL) and with saturated brine (50 mL) each three times. The aqueous layer was extracted with 50 mL of diethyl ether three times, combined with the previously obtained ether layer, dried over magnesium sulfate, filtered and concentrated. The residue was purified by flash column chromatography (ethyl acetate/hexane 1:4) to give Compound 5a (2.2 g, 58%) and Compound 5b (1.22 g, 32%) as colorless oily alcohols. 5a: [α]²⁰ _{D} 19.56 (c 1.23, CHCl₃); ¹H NMR (400 MHz, CDCl₃) δ 1.20 (s, 9H), 2.26 (d, 1H, J= 11.6 Hz), 4.18 (dd, 1H, J = 4.8, 12.0 Hz), 4.60 (d, 1H, J= 12.0 Hz), 4.73 (ddt, 1H, J= 2.4, 3.4, 4.8 Hz), 4.81 (d, 1H, J= 12.0 Hz), 5.15 (d, 1H, J= 4.4 Hz), 5.18 (t, 1H, J= 2.4 Hz), 5.39 (t, 1H, J= 2.4 Hz), 7.34 (m, .5H). 5b: [α]²⁰ _{D}-4.00 (c 1.07, CHCl₃: ¹H NMR (400 MHz, CDCl₃) δ 1.27 (s, 9H), 2.61 (bs, 1H), 4.14 (dd, 1H, J = 6.8, 12.0 Hz), 4.19 (dd, 1H, J= 4.8, 12.0 Hz), 4.42 (t, 1H, J= 1.2 Hz), 4.49 (d, 1H, J= 11.6 Hz), 4.76 (d, 1H, J= 11.6 Hz), 4.89 (qt, 1H, J= 1.2, 4.8, 6.2 Hz), 5.03 (s, 1H), 5.26 (t, 1H, J= 1.2 Hz), 7.33 (m, 5H).

### (5) Synthesis of benzyl 5-O-pivaloyl-3-deoxy-3-C-methylene-α-D-xylo-pentofuranoside (Compound 6)

Compound 5a (2.0 g, 6.25 mmol), p-nitrobenzoic acid (2.1 g, 12,6 mmol) and triphenylphosphine (3. 3 g, 12.6 mmol) were dissolved in THF (40 mL), cooled to 0°C under argon atmosphere, and a 40% DEAD-toluene solution (5.8 g, 13.9 mmol) was added. The reaction mixture was stirred at that temperature for 15 min., warmed to room temperature and stirred for further 3 hours. The reaction mixture was cooled to 0°C, then MeOH (10 mL) was added. The mixture was diluted with diethyl ether (100 mL) and washed with saturated aqueous sodium bicarbonate solution (50 mL) and saturated brine (50 mL), each three times. The aqueous layer was extracted with 50 mL of diethyl ether three times, combined with the previously obtained ether layer. The ether layer was dried over magnesium sulfate, filtered and concentrated. The residue was purified by flash column chromatography (ethyl acetate/hexane 1:10) to give a crude ester compound as a colorless oil, which was subjected to the next reaction without further purification.

The ester was dissolved in MeOH (100 mL), cooled to 0°C, and 1.0M aqueous sodium hydroxide solution (1.0 mL, 1.00 mmol) was added dropwise. The reaction mixture was stirred for 1 hour at the same temperature, neutralized by the addition of 1.0 M hydrochloric acid (1.5 mL, 1.5 mmol) and concentrated. The residue was dissolved in diethyl ether (200 mL) and washed with a saturated aqueous sodium bicarbonate solution (50 mL) and with saturated brine (50 mL) each three times. The aqueous layer was extracted with 50 mL of diethyl ether three times, combined with the previously obtained ether layer. The ether layer was dried over magnesium sulfate, filtered and concentrated. The residue was purified by silica gel chromatography (ethyl acetate/hexane 1:5) to give alcohol Compound 6 (1.67 g, 84%) as a colorless oil.
[α]²⁰ _{D} 11.18 (c 1.00, CHCl₃) ; ¹H NMR (400 MHz, CDCl₃ δ 1.22 (s, 9H), 2.09 (d, 1H, J= 7.6 Hz), 4.25 (dd, 1H, J= 4.8, 12.4 Hz), 4.33 (dd, 1H, J= 3.2, 12.4 Hz), 4.37 (d, 1H, J= 7.6 Hz), 4.55 (d, 1H, J= 11.6 Hz), 4.73 (ddd, 1H, J= 1.6, 3.2, 4.3 Hz), 4.74 (d, 1H, J= 11.6 Hz), 5.10 (s, 1H), 5.26 (s, 1H), 5.54 (t, 1H, J= 1.6 Hz), 7.33 (m, 5H).

### (6) Synthesis of benzyl 5-O-pivaloyl-2-methoxymethyl-3-deoxy-3-C-methylene-α-D-xylofuranose (Compound 7)

Compound 6 (1.0 g, 3.13 mmol) was dissolved in dichloromethane (25 mL), to which diisopropylethylamine (1.24 g, 9.61 mmol) and chloromethyl ethyl ether (1.29 g, 16.0 mmol) were added dropwise at 0°C. Tetrabutylammonium iodide (360 mg, 975 µmol) was added to the reaction mixture and then the mixture was stirred for 14 hours at room temperature in a dark. After the completion of the reaction, the reaction mixture was diluted with diethyl ether (150 mL) and washed with saturated aqueous ammonium chloride solution (25 mL) and with saturated brine (25 mL), each three times. The organic layer was dried over magnesium sulfate, filtered and concentrated. The residue was purified by silica gel chromatography (ethyl acetate/hexane 1:9) to give Compound 7 (1.01 g, 88%) as a colorless oil. ¹H NMR (400 MHz, CDCl₃) δ 1.23 (s, 9H), 3.35 (s, 3H), 4.18 (dd, 1H, J= 6.4, 11.2 Hz), 4.22 (dd, 1H, J= 5.2, 11.2 Hz), 4.37 (s, 1H), 4.55 (d, 1H, J= 12.0 Hz), 4.60 (d, 1H, J= 6.8 Hz), 4.69 (ddd, 1H, J= 1.6, 5.2, 6.4 Hz), 4.75 (d, 1H, J= 12.0 Hz), 4.76 (d, 1H, J= 6.8 Hz), 5.21 (s, 1H), 5.36 (s, 1H), 5.46 (d, 1H, J= 1.6 Hz), 7.33 (m, 5H).

### (7) Synthesis of benzyl 5-O-pivaloyl-2-methoxymethyl-3-deoxy-3-C-hydroxymethyl-α-D-xylo-pentofuranose (Compound 8)

Compound 7 (2.0 g, 5.49 mmol) was dissolved in THF (20 mL), cooled to 0°C, and a THF solution of 0.5M 9-BBN (9-borabicyclo[3,3,1]nonane) (20 mL, 10.0 mmol) was added dropwise. The reaction mixture was heated to 50°C, stirred for 3 hours and cooled to 0°C. To the mixture, 3.0M sodium hydroxide solution (6.4 mL, 19.2 mmol) and 30% hydrogen peroxide solution (12.8 mL) were added, followed by stirring vigorously for 2 hours at room temperature. The mixture was diluted with ethyl acetate (200 mL) and washed with water (50 mL), 5% aqueous sodium sulfite solution (50 mL) and with brine (50 mL), each three times. The thus obtained aqueous layer was extracted with 50 mL of diethyl ether three times. The ether layer was combined with the previously obtained organic layer, dried over magnesium sulfate, filtered and concentrated. The thus obtained residue was purified by silica gel chromatography (ethyl acetate/hexane 1:3) to give Compound 8 (1.78 g, 85%) as a colorless oil. [α]²⁰ _{D} 7.06 (c 1.70, CHCl₃); ¹H NMR (400 MHz, CDCl₃ δ 1.22 (s, 9H), 2.34 (t, 1H, J= 6.0 Hz), 2.89 (tt, 1H, J= 6.0, 8.4 Hz), 3.37 (s, 3H), 3.86 (dt, 1H, J= 6.0, 11.2 Hz), 3.88 (ddd, 1H, J= 6.0, 8.4, 11.2 Hz), 4.19 (dd, 1H, J= 7.2, 11.6 Hz), 4.20 (d, 1H, J= 6.0 Hz), 4.23 (dd, 1H, J= 5.6, 11.6 Hz), 4.38 (dd, 1H, J= 5.6, 7.2, 8.4 Hz),4.50 (d, 1H, J= 12.0 Hz), 4.66 (s, 2H), 4.73 (d, 1H, J= 12.0 Hz), 5.15 (s, 1H), 7.34 (m, 5H).

### (8) Synthesis of benzyl 5-O-pivaloyl-2-methoxymethyl-3-deoxy-3-C-(tert-butyldimethylsilyloxymethyl)-α-D-xylo-pentofuranose (Compound 9)

Compound 8 (2.97 g, 7.77 mmol) was dissolved in DMF (30 mL), to which solution imidazole (1.06 g, 15.6 mmol) and TBDMSC1 (tert-butyldimethylsilyl chloride) (1.76 g, 11.7 mmol) were added at room temperature. The reaction mixture was stirred for 3 hours at room temperature, diluted with diethyl ether (200 mL) and washed with water (25 mL) and with saturated brine (25 mL), each three times. The thus obtained aqueous layer was extracted with 25 mL of diethyl ether three times. The ether layer was combined with the previously obtained organic layer, dried over magnesium sulfate, filtered and concentrated. The residue was purified by silica gel chromatography (ethyl acetate/hexane 1:9) to give Compound 9 (3.82 g, 99%) as a colorless oil.
[α]²⁰ _{D} 7.65 (c 1.00, CHCl₃); ¹H NMR (400 MHz, CDCl₃) δ 0.02 (s, 3H), 0.03 (s, 3H), 0.83 (s, 9H), 1.18 (s, 9H), 2.80 (qt, 1H, J= 4.8, 6.8, 8.8 Hz), 3.28 (s, 3H), 3.70 (dd, 1H, J= 6.8, 10.0 Hz), 3.76 (dd, 1H, J= 8.8, 10.0 Hz), 4.07 (d, 1H, J= 4.8 Hz), 4.09 (dd, 1H, J= 7.4, 12.0 Hz), 4.13 (dd, 1H, J= 4.8, 12.0 Hz), 4.69 (d, 1H, J= 12.0 Hz), 5.10 (s, 1H), 7.28 (m, 5H); HREIMS C₂₅H₄₁O₆Si (M⁺-OCH₃) calcd. 465.2673, found 465.2666.

### (9) Synthesis of 3S,4R,5R-4-(tert-butyldimethylsilyloxymethyl)-3-methoxymethyloxy-6-O-pivaloyl-hex-1-en-5-ol (Compound 10)

Compound 9 (3.98 g, 8.02 mmol) was dissolved in ethanol (40 mL), and Pd(OH)₂ (400 mg) was added, followed by stirring under hydrogen atmosphere for 12 hours at room temperature for catalytic reduction. The reaction mixture was filtered through CELITE, and the filtrate was concentrated to give hemiacetal compound as a colorless oil which was subjected to the next reaction without further purification.

Methyltriphosphonium bromide (10.9 g, 30.5 mL) was dissolved in THF (50 mL), cooled to 0°C under argon atmosphere, and 1.0 M LiHMDS (lithium bis(trimethylsilyl)amide) in THF (29 mL, 29.0 mmol) was added dropwise. The mixture was stirred at room temperature for 40 min. and then cooled again to 0°C when the color of the mixture was changed to yellow, followed by the addition of a THF solution of the hemiacetal (20 mL). The reaction mixture was stirred for 40 min. at 0°C, then MeOH (5 mL) was added. The mixture was diluted with diethyl ether (300 mL) and washed with saturated aqueous ammonium chloride solution (50 mL) and with saturated brine (50 mL), each three times. The organic layer was collected. The aqueous layer was extracted with 50 mL of diethyl ether three times. The ether layers were combined with the previously obtained ether layer, dried over magnesium sulfate, filtered and concentrated. The residue was purified by flash column chromatography (ethyl acetate/hexane 1:6) to give olefin Compound 10 (2.57 g, 79%) as a colorless oil.
[α]²⁰ _{D}-5.63 (c 1.00, CHCl₃); ¹H NMR (400 MHz, CDCl₃) δ 0.05 (s, 3H), 0.07 (s, 3H), 0.89 (s, 9H), 1.21 (s, 9H), 2.74 (ddt, 1H, J= 4.4, 7.2, 8.0 Hz), 3.40 (s, 3H), 3.54 (d, 1H, J= 5.6 Hz), 3.82 (dd, 1H, J= 4.4, 10.4 Hz), 3.90 (dd, 1H, J= 4.8, 5.6, 7.2 Hz), 4.40 (t, 1H, J= 8.0 Hz), 4.58 (d, 1H, J= 6.4 Hz), 4.72 (d, 1H, J= 6.4 Hz), 5.30 (dd, 1H, J= 1.2, 16.8 Hz), 5.33 (dd, 1H, J= 1.2, 10.4 Hz), 5.70 (ddd, 1H, J= 8.0, 10.4, 16.8 Hz); HREIMS C₁₉H₃,O₅Si (M⁺-OCH₃) calcd. 373.2411, found 373.2421.

### (10) Synthesis of 3S,4R,5R-4-(tert-butyldimethylsilyloxymethyl)-3-methoxymethyloxy-hex-1-en-5,6-diol (Compound 11)

Compound 10 (3.20 g, 7.92 mmol) was dissolved in dichloromethane (50 mL), and 1.0M DIBAL (diisobutyl aluminum hydride) in toluene (19.8 mL, 19.8 mmol) was added dropwise over 30 min. After 10 min., methanol (1 mL) and saturated aqueous ammonium chloride solution (1 mL) were added to the reaction mixture, which was then diluted with diethyl ether (250 mL) and filtered through CELITE. The filtrate was washed with a saturated aqueous ammonium chloride solution (50 mL) and with saturated brine (50 mL), each three times. The organic layer was collected. The aqueous layer was extracted with 25 mL of diethyl ether three times. The ether layers were combined with the previously obtained ether layer, dried over magnesium sulfate, filtered and concentrated. The residue was purified by flash column chromatography (ethyl acetate/hexane 2:3) to give diol Compound 11 (2.12 g, 84%) as a colorless oil.
[α]²⁰ _{D}-6.89 (c 1.00, CHCl₃); ¹H NMR (400 MHz, CDCl₃) δ 0.05 (s, 3H), 0.06 (s, 3H), 0.89 (s, 9H), 1.77 (ddt, 1H, J= 4.8, 6.5, 8.0 Hz), 2.67 (dd, 1H, J= 4.8, 8.4 Hz), 3.40(s, 3H), 3.52 (d, 1H, J= 6.0 Hz), 3.70(m, 1H), 3.83 (d, 1H, J= 4.8 Hz), 4.14 (m, 1H), 4.30 (t, 1H, J= 8.0 Hz), 4.57 (d, 1H, J= 6.8 Hz), 4.71 (d, 1H, J= 6.8 Hz), 5.29 (d, 1H, J= 16.8 Hz), 5.32 (dd, 1H, J= 10.0 Hz), 5.69 (ddd, 1H, J= 8.0, 10.0, 16.8 Hz); HREIMS C₁₄H₂₉O₄Si (M⁺OCH₃) calcd. 289.1835, found 289.1838.

### (11) Synthesis of 3S,4R,5R-4-(tert-butyldimethylsilyloxymethyl)-3-methoxymethyloxy-hex-1-en-5,6-epoxide (Compound 12)

Compound 11 (3.20 g, 11.6 mmol) was dissolved in dichloromethane (40 mL), mixed with DMAP (4-(dimethylamino)pyridine) (2.84 g, 23.4 mmol), cooled to 0°C and vigorously stirred. To the mixture, 2-mesitylenesulfonyl chloride (3.80 g, 17.4 mmol) was slowly added, followed by stirring at that temperature for 4 hours. The reaction mixture was diluted with diethyl ether (200 mL) and washed with water (25 mL) and with saturated brine (25 mL), each three times. The aqueous layer was extracted with 25 mL of diethyl ether three times. The ether layers were combined with the previously obtained ether layer, dried over magnesium sulfate, filtered and concentrated to give a sulfonate, which was used to the next step without further purification.

The sulfonate was dissolved in THF (50 mL), cooled to -78°C, 1.0 M LiHMDS in THF (2.5 mL, 2.5 mmol) was added dropwise. After stirring for 20 min. at that temperature, the mixture was warmed to 0°C and stirred for further 20 min. The reaction mixture was diluted with diethyl ether (200 mL), washed with a saturated aqueous ammonium chloride solution (25 mL) and with saturated brine (25 mL), each three times. The aqueous layer was extracted with 25 mL of diethyl ether three time. The ether layers were combined with the previously obtained diethyl ether layer, dried over magnesium sulfate, filtered and concentrated. The residue was purified by flash column chromatography (ethyl acetate/hexane 1:9) to give epoxy Compound 12 (3.01 g, 86%) as a colorless oil.
[α]²⁰ _{D}-7.90 (c 1.00, CHCl₃); ¹H NMR (400 MHz, CDCl₃) δ 0.02 (s, 3H), 0.04 (s, 3H), 0.87 (s, 9H), 1.26 (dddd, 1H, J= 3.4, 4.0, 5.2, 7.2 Hz), 2.62 (dd, 1H, J= 2.8, 5.2 Hz), 2.87 (t, 1H, J= 5.2 Hz), 3.04 (td, 1H, J= 2.8, 3.4, 5.2 Hz), 3.35 (s, 3H), 3.70 (dd, 1H, J= 4.0, 10.0 Hz), 3.82 (dd, 1H, J= 5.2, 10.0 Hz), 4.31 (t, 1H, J= 7.2 Hz), 4.51 (d, 1H, J= 6.4 Hz), 4.68 (d, 1H, J= 6.4 Hz), 5.24 (d, 1H, J= 10.2 Hz), 5.25 (d, 1H, J= 17.2 Hz), 5.70 (ddd, 1H, J= 7.2, 10.0, 17.2 Hz); HREIMS C₁₄H₂₇O₃Si (M⁺-OCH₃) calcd. 271.1729, found 271.1732.

### (12) Synthesis of 3S,4R,5R-4-(tert-butyldimethylsilyloxymethyl)-3-methoxymethyloxy-8-trimethylsilyl-oct-1-en-7-yn-5-ol (Compound 13)

Ethynyltrimethylsilane (3.88 g, 39.6 mmol) was dissolved in THF (100 mL), cooled to 0°C. 1.54M n-Butyl lithium-hexane (22.7 mL, 36.0 mmol) was slowly added. The mixture was stirred at that temperature for 15 min., then cooled to -78°C. A solution of Compound 12 (3.01 g, 9.97 mmol) in THF (20 mL) and boron trifluoride diethyl ether complex (1.70 g, 12.0 mmol) were added. The mixture was warmed to room temperature, stirred for 40 min., diluted with diethyl ether (300 mL) and washed with a saturated aqueous ammonium chloride solution (50 mL) and with saturated brine (50 mL), each three times. The aqueous layer was extracted with diethyl ether (50 mL) three times. The ether layers were combined with the previously obtained ether layer, dried over magnesium sulfate, filtered and concentrated. The residue was purified by flash column chromatography (ethyl acetate/hexane 1:9) to give enyne Compound 13 (3.20 g, 80%) as a colorless oil.
[α]²⁰ _{D}- 7.14 (c 1.00, CHCl₃); ¹H NMR (400 MHz, CDCl₃) δ 0.04 (s, 3H), 0.05 (s, 3H), 0.11 (s, 9H), 0.87 (s, 9H), 1.93 (ddt, 1H, J= 4.0, 5.6, 8.0 Hz), 2.46 (dd, 1H, J= 8.0, 16.8 Hz), 2.63 (dd, 1H, J= 6.4, 16.8 Hz), 3.38 (s, 3H), 3.60 (d, 1H, J= 5.6 Hz), 3.81 (dd, 1H, J= 4.0, 10.0 Hz), 3.92 (dd, 1H, J= 5.6, 10.8 Hz), 4.21 (ddt, 1H, J= 5.6, 6.4, 8.0 Hz), 4.44 (t, 1H, J= 8.0 Hz), 4.58 (d, 1H, J= 6.4 Hz), 4.70 (d, 1H, J= 6.4 Hz), 5.28 (d, 1H, J= 17.2 Hz), 5.29 (d, 1H, J= 10.8 Hz), 5.71 (td, 1H, J= 8.0, 10.8, 17.2 Hz).

### (13) Synthesis of 3S,4R,5R-4-hydroxymethyl-3-methoxymethyloxy-oct-1-en-7-yn-5-ol (Compound 14)

Compound 13 (3.20 g, 8.0 mmol) was dissolved in THF (45 mL), cooled to 0°C, and 1.0M TBAF (tetrabutylammonium fluoride) in THF (17.6 mL, 17.6 mmol) was added. The reaction mixture was warmed to room temperature, stirred for further 1 hour, diluted with ethyl acetate (200 mL) and washed with a saturated aqueous ammonium chloride solution (25 mL) and with saturated brine (25 mL), each three times. The aqueous layer was extracted with ethyl acetate (25 mL) three times. The ethylacetate layers were combined with the previously obtained organic layer, dried over magnesium sulfate, filtered and concentrated. The residue was purified by flash column chromatography (ethyl acetate/hexane 2:3) to give diol Compound 14 (1.69 g, 99%) as a colorless oil.
[α]²⁰ _{D}-11.15 (c 1.00, CHCl₃); ¹H NMR (400 MHz, CDCl₃) δ 1.86 (ddt, 1H, J= 3.2, 4.8, 7.2 Hz), 2.05 (t, 1H, J= 2.8 Hz), 2.50 (ddd, 1H, J= 2.8, 6.8, 16.8 Hz), 2.60 (bs, 1H), 2.63 (ddd, 1H, J= 2.8, 6.8, 16.8 Hz), 3.32 (d, 1H, J= 4.4 Hz), 3.34 (s, 3H), 3.83 (dd, 1H, J= 4.8, 11.2 Hz), 4.00 (dd, 1H, J= 4.8, 11.2 Hz), 4.30 (ddt, 1H, J= 3.2, 4.4, 6.8 Hz), 4.44 (tt, 1H, J= 1.2, 7.2 Hz), 4.61 (d, 1H, J= 6.8 Hz), 4.70 (d, 1H, J= 6.8 Hz), 5.33 (dt, 1H, J= 1.2, 10.4 Hz), 5.35 (d, 1H, J= 1.2, 17.2 Hz), 5.71 (ddd, 1H, J=7.2, 10.4, 17.2 Hz).

### (14) Synthesis of 3S,4R,5R-4-pivaloyloxymethyl-3-methoxymethyloxy-oct-1-en-7-yn-5-ol (Compound 15)

Compound 14 (1.62 g, 7.57 mmol) was dissolved in pyridine (1.7 mL) and dichloromethane (6.8 mL) and cooled to 0°C. To the solution, trimethylacetyl chloride (1.08 g, 8.96 mmol) was added dropwise over 30 min., and stirred at the same temperature for 1 hour. The reaction mixture was warmed to room temperature, stirred for 4 hours, diluted with diethyl ether (100 mL) and washed with saturated aqueous ammonium chloride solution (20 mL) and with saturated brine (20 mL), each three times. The aqueous layer was extracted with diethyl ether (20 mL) three times. The ether layers were combined with the previously obtained ether layer, dried over magnesium sulfate, filtered and concentrated. The residue was purified by flash column chromatography (ethyl acetate/hexane 1:4) to give alcohol Compound 15 (1.92 g, 85%) as a colorless oil.
[α]²⁰ _{D}-10.05 (c 1.00, CHCl₃); ¹H NMR (400 MHz, CDCl₃) δ 1.22 (s, 9H), 2.03 (t, 1H, J= 8.0 Hz), 2.46 (ddt, 1H, J= 2.4, 4.8, 5.6 Hz), 2.46 (ddd, 1H, J= 2.4, 7.6, 16.8 Hz), 2.58 (ddd, 1H, J= 2.4, 6.4, 16.8 Hz), 3.10 (d, 1H, J= 2.4 Hz), 3.39 (s, 3H), 4.26 (ddt, 1H, J= 2.4, 6.4, 7.6 Hz), 4.29 (dd, 1H, J= 5.6, 11.6 Hz), 4.33 (dd, 1H, J= 5.6, 11.6 Hz), 4.40 (ddt, 1H, J = 1.2, 4.8, 6.8 Hz), 4.44 (t, 1H, J= 8.0 Hz), 4.57 (d, 1H, J= 7.2 Hz), 4.70 (d, 1H, J= 7.2 Hz), 5.31 (dt, 1H, J= 1.2, 17.6 Hz), 5.34 (d, 1H, J= 1.2, 10.4 Hz), 5.71 (ddd, 1H, J= 6.8, 10.4, 17.6 Hz).

### (15) Synthesis of 3S,4R,5R-4-pivaloyloxymethyl-oct-1-en-7-yn-3,5-diol (Compound 16)

Compound 15 (2.10 g, 7.05 mmol) was dissolved in tert-butyl alcohol (60 mL). Pyridinium p-toluenesulfonate (17.6 g, 70.0 mmol) was added. The mixture was refluxed for 12 hours. The reaction mixture was cooled to room temperature and then concentrated. The residue was diluted with diethyl ether (200 mL) and washed with saturated sodium bicarbonate solution (20 mL) and with saturated brine (20 mL), each three times. The aqueous layer was extracted with diethyl ether (20 mL) three times. The ether layers were combined with the previously obtained ether layer, dried over magnesium sulfate, filtered and concentrated. The residue was purified by flash column chromatography (ethyl acetate/hexane 1:3) to give diol Compound 16 (1.33 g, 74%) as a colorless oil.
[α]²⁰ _{D} 1.75 (c 1.00, CHCl₃) ; ¹H NMR (400 MHz, CDCl₃) δ 1.22 (s, 9H), 2.05 (t, 1H, J= 2.4 Hz), 2.06 (dddd, 1H, J= 2.4, 4.4, 5.6, 6.8 Hz), 2.43 (ddd, 1H, J= 2.4, 7.2, 16.8 Hz), 2.57 (ddd, 1H, J= 2.4, 7.2, 16.8 Hz), 2.73 (d, 1H, J= 4.4 Hz), 3.07 (d, 1H, J= 2.4 Hz), 4.24 (tt, 1H, J= 2.4, 7.2 Hz), 4.31 (dd, 1H, J= 5.6, 11.6 Hz), 4.43 (dd, 1H, J= 6.8, 11.6 Hz), 4.45 (ddt, 1H, J= 1.2, 4.4, 5.6 Hz), 5.28 (dt, 1H, J= 1.2, 10.4 Hz), 5.28 (dt, 1H, J= 1.2, 10.4 Hz), 5.40 (d, 1H, J= 1.2, 16.8 Hz), 5.92 (ddd, 1H, J= 5.6, 10.4, 16.8 Hz).

### (16) Synthesis of 3S,4R,5R-4-pivaloyloxymethyl-3,5-di-(tert-butyldimethylsilyloxy)-oct-1-en-7-yne (Compound 17)

Compound 16 (1.31 g, 5.16 mmol) was dissolved in dichloromethane (25 mL), cooled to 0°C, and 2,6-lutidine (2.21 g, 20.6 mmol) and TBDMSOTf (tert-butyldimethylsilyltriflate) (4.09 g, 15.5 mmol) were added. The reaction mixture was stirred for 2 hours at the same temperature, diluted with diethyl ether (150 mL) and washed with a saturated aqueous sodium bicarbonate solution (20 mL) and with saturated brine (20 mL), each three times. The aqueous layer was extracted with diethyl ether (20 mL) three times. The ether layers were combined with the previously obtained ether layer, dried over magnesium sulfate, filtered and concentrated. The residue was purified by flash column chromatography (ethyl acetate/hexane 1:40) to give silyl ether Compound 17 (2.47 g, 99%) as a colorless oil.
[α]²⁰ _{D}-0.37 (c 1.00, CHCl₃); ¹H NMR (400 MHz, CDCl₃) δ 0.04 (s, 3H), 0.06 (s, 3H), 0.07 (s, 3H), 0.10 (s, 3H), 0.88 (s, 9H), 0.89 (s, 9H), 1.20 (s, 9H), 1.98 (t, 1H, J= 2.4 Hz), 2.19 (dq, 1H, J= 4.0, 6.0 Hz), 2.45 (ddd, 1H, J= 2.4, 4.8, 16.4 Hz), 2.54 (ddd, 1H, J= 2.4, 7.2, 16.4 Hz), 3.99 (dd, 1H, J= 6.0, 12.0 Hz), 4.16 (ddd, 1H, J= 4.0, 4.8, 7.2 Hz), 4.17 (dd, 1H, J = 6.0, 7.6 Hz), 4.25 (dd, 1H, J= 6.0, 12.0 Hz), 5.09 (d, 1H, J= 10.0 Hz), 5.16 (d, 1H, J = 17.2 Hz), 5.84 (ddd, 1H, J= 7.6, 10.0, 17.2 Hz); HREIMS C₂₂H₄₁OSi₂ (M⁺-t-Bu) calcd. 425.2543, found 425.2543

### (17) Synthesis of 3S,4R,5R-4-hydroxymethyl-3,5-di-(tert-butyldimethylsilyloxy)-oct-1-en-7-yne (Compound 18)

Compound 17 (2.47 g, 5.12 mmol) was dissolved in dichloromethane (20 mL) and cooled to -78°C. 1.0M Diisobutyl aluminium hydride in toluene (7.7 mL, 7.70 mmol) was added dropwise over 30 min. The mixture was stirred at the same temperature for 10 min. Methanol (1 mL) and saturated aqueous ammonium chloride solution (1 mL) were added, and diluted with diethyl ether (200 mL). The mixture was filtered through CELITE. The filtrate was washed with saturated aqueous ammonium chloride solution (20 mL) and with saturated brine (20 mL), each three times. The aqueous layer was extracted with diethyl ether (20 mL) three times. The ether layers were combined with the previously obtained ether layer, dried over magnesium sulfate, filtered and concentrated. The residue was purified by flash column chromatography (ethyl acetate/hexane 1:10) to give alcohol Compound 18 (1.96 g, 96%) as a colorless oil.
[α]²⁰ _{D} 0.11 (c 1.00, CHCl₃); ¹H NMR (400 MHz, CDCl₃) δ 0.06 (s, 3H), 0.10 (s, 3H), 0.11 (s, 3H), 0.13 (s, 3H), 0.90 (s, 18H), 2.01 (t, 1H, J= 2.8 Hz), 2.09 (ddt, 1H, J= 2.0, 4.8, 7.2 Hz), 2.46 (ddd, 1H, J= 2.8, 4.8, 16.8 Hz), 2.51 (ddd, 1H, J= 2.8, 6.8, 16.8 Hz), 3.07 (dd, 1H, J= 4.8, 7.2 Hz), 3.72 (ddd, 1H, J= 4.8, 7.2, 12.4 Hz), 3.82 (dt, 1H, J = 7.2, 12.4 Hz), 4.08 (dt, 1H, J= 4.8, 6.8 Hz), 4.33 (dd, 1H, J= 6.0, 12.0 Hz), 5.20 (dt, 1H, J = 2.0, 10.4 Hz), 5.28 (dt, 1H, J = 2.0, 17.2 Hz), 5.84 (ddd, 1H, J= 7.2, 10.4, 17.2 Hz); HREIMS C₂₁H₁₂O₃Si₂ (M⁺) calcd. 398.2672, found 398.2669.

### (18) Synthesis of 3S,4R,5R-4-(tert-butyldimethylsilyloxymethyl)-3,5-di-(tert-butyldimethylsilyloxy)-oct-1-en-7-yne (Compound 19)

Compound 18 (40.0 mg, 101 µmol) was dissolved in DMF (2.0 mL), and imidazole (13.6 mg, 200 µmol) and TBDMSCl (tert-butyldimethylsilyl chloride) (22.6 mg, 150 µL) were added at room temperature. The mixture was stirred for 3 hours. The reaction mixture was diluted with diethyl ether (20 mL) and washed with water (5 mL) and with saturated brine (5 mL), each three times. The aqueous layer was extracted with diethyl ether (5 mL) three times. The ether layers were combined with the previously obtained ether layer, dried over magnesium sulfate, filtered and concentrated. The residue was purified by flash column chromatography (ethyl acetate/hexane 1:50) to give silyl ether Compound 19 (46.0 mg, 88%) as a colorless oil.
[α]²⁰ _{D} 0.59 (c 1.00, CHCl₃); ¹H NMR (400 MHz, CDCl₃) δ 0.03 (s, 3H), 0.04 (s, 3H), 0.06 (s, 3H), 0.10 (s, 3H), 0.89 (s, 9H), 0.90 (s, 18H), 1.93 (t, 1H, J= 2.8 Hz), 2.00 (ddt, 1H, J= 5.2, 5.6, 6.4 Hz), 2.44 (ddd, 1H, J= 2.8, 5.6, 16.8 Hz), 2.57 (ddd, 1H, J= 2.8, 5.6, 16.8 Hz), 3.56 (dd, 1H, J= 6.0, 10.0 Hz), 3.81 (dd, 1H, J= 6.0, 10.0 Hz), 4.09 (dt, 1H, J = 4.8, 5.6 Hz), 4.30 (ddt, 1H, J= 1.2, 5.2, 6.8 Hz), 5.03 (dt, 1H, J= 1.2, 9.6 Hz), 5.11 (dt, 1H, J = 1.2, 17.2 Hz), 5.92 (ddd, 1H, J= 6.8, 9.6, 17.2 Hz).

### (19) Synthesis of (3S,4R,5R-4-cyanomethyl-3,5-di-(tert-butyldimethylsilyloxy)-oct-1-en-7-yne (Compound 20)

Compound 18 (314 mg, 789 µmol) was dissolved in dichloromethane, and DMAP (dimethylaminopyridine) (372 mg, 3.04 mmol) was added, followed by cooling to 0°C. 2-Mesitylenesulfonyl chloride (582 mg, 2.66 mml) was added with vigorous stirring. The mixture was stirred for 12 hours at the same temperature. The reaction mixture was diluted with diethyl ether (100 mL) and washed with water (15 mL) and with saturated brine (15 mL), each three times. The aqueous layer was extracted with diethyl ether (15 mL) three times. The ether layers were combined with the previously obtained ether layer, dried over magnesium sulfate, filtered and concentrated. The thus obtained residue was subjected to the next step without further purification.

The sulfon ester was dissolved in DMSO (dimethylsulfoxide) (5.0 mL), and sodium cyanide (78.0 mg, 1.59 mmol) was added, followed by stirring at 70°C for 2 hours. The reaction mixture was cooled to room temperature, diluted with diethyl ether (100 mL) and washed with water (15 mL) and with saturated brine (15 mL), each three times. The aqueous layer was extracted with diethyl ether (15 mL) three times. The ether layers were combined with the previously obtained ether layer, dried over magnesium sulfate, filtered and concentrated. The residue was purified by flash column chromatography (ethyl acetate/hexane 1:20) to give nitrile Compound 20 (263 mg, 82%) as a colorless oil. [α]²⁰ _{D}-0.44 (c 1.00, CHCl₃); ¹H NMR (400 MHz, CDCl₃) δ 0.06 (s, 3H), 0.09 (s, 3H), 0.12 (s, 3H), 0.13 (s, 3H), 0.90 (s, 9H), 0.91 (s, 9H), 2.03 (t, 1H, J= 2.8 Hz), 2.29 (tt, 1H, J= 6.0, 6.8 Hz), 2.36 (dd, 1H, J= 6.0, 16.4 Hz), 2.46 (ddd, 1H, J= 2.8, 4.4, 16.8 Hz), 2.48 (dd, 1H, J= 6.0, 16.4 Hz), 2.52 (ddd, 1H, J= 2.8, 6.8, 16.8 Hz), 4.04 (dt, 1H, J = 4.4, 6.8 Hz), 4.20 (ddt, 1H, J= 1.2, 6.4, 6.8 Hz), 5.24 (dt, 1H, J= 1.2, 10.4 Hz), 5.31 (dt, 1H, J = 1.2, 16.8 Hz), 5.82 (ddd, 1H, J= 6.4, 10.4, 16.8 Hz).

### (20) Synthesis of 3S,4R,5R-3,5-di-(tert-butyldimethylsilyloxy)-4-(2'-ethanal)-oct-1-en-7-yne (Compound 21)

Compound 20 (184 mg, 452 µmol) was dissolved in dichloromethane (2.0 mL), cooled to -78°C, and 1.0M diisobutyl aluminium hydride in toluene (530 µL, 530 µmol) was added over 10 min., followed by stirring for 1 hour at that temperature. Methanol (1 mL) and a saturated aqueous ammonium chloride solution (1 mL) were added to the reaction mixture, which was then diluted with diethyl ether (100 mL). The mixture was filtered through CELITE. The filtrate was washed with saturated aqueous ammonium chloride solution (10 mL) and saturated brine (10 mL), each three times. The aqueous layer was extracted with diethyl ether (10 mL) three times. The ether layers were combined with the previously obtained ether layer, dried over magnesium sulfate, filtered and concentrated. The residue was purified by flash column chromatography (ethyl acetate/hexane 1:20) to give aldehyde Compound 21 (157 mg, 85%) as a colorless oil.
[α]²⁰ _{D} 0.77 (c 1.00, CHCl₃); ¹H NMR (400 MHz, CDCl₃) δ 0.04 (s, 3H), 0.06 (s, 3H), 0.08 (s, 3H), 0.09 (s, 3H), 0.88 (s, 9H), 0.89 (s, 9H), 2.04 (t, 1H, J= 2.8 Hz), 2.37 (ddd, 1H, J= 2.8, 6.0, 16.8 Hz), 2.40 (ddd, 1H, J= 2.8, 6.4, 17.2 Hz), 2.42 (ddd, 1H, J= 1.6, 7.2, 17.2 Hz), 2.44 (ddd, 1H, J= 2.8, 6.0, 16.8 Hz), 2.68 (ddd, 1H, J= 5.2, 6.4 Hz), 3.84 (dt, 1H, J = 5.2, 6.0 Hz), 4.23 (ddt, 1H, J= 1.6, 6.4, 6.8 Hz), 5.18 (dt, 1H, J= 1.6, 10.0 Hz), 5.21 (dt, 1H, J = 1.6, 16.0 Hz), 5.73 (ddd, 1H, J = 6.8, 10.0, 16.8 Hz); HREIMS C₂₂H₄₂O₃Si₂ (M⁺) calcd. 410.2673, found 410.2667.

### (21) Synthesis of 3S,4R,5R-4-(2'-hydroxyethyl)-3,5-di-(tert-butyldimethylsilyloxy)-oct-1-en-7-yne (Compound 22)

Compound 21 (157 mg, 383 µmol) was dissolved in methanol (2.0 mL), cooled to 0°C, and sodium borohydride (28.0 mg, 741 µmol) was added, followed by stirring at the same temperature for 30 min. The reaction mixture was warmed to room temperature, diluted with diethyl ether (100 mL) and washed with water (10 mL) and with saturated brine (10 mL), each three times. The aqueous layer was extracted with diethyl ether (10 mL) three times. The ether layers were combined with the previously obtained ether layer, dried over magnesium sulfate, filtered and concentrated. The residue was purified by flash column chromatography (ethyl acetate/hexane 1:9) to give alcohol Compound 22 (129 mg, 82%) as a colorless oil.
[α]²⁰ _{D} 1.40 (c 1.00, CHCl₃): ¹H NMR (400 MHz, CDCl₃) δ 0.08 (s, 6H), 0.10 (s, 6H), 0.90 (s, 9H), 0.91 (s, 9H), 1.67 (m, 2H), 2.03 (t, 1H, J= 2.8 Hz), 2.29 (tt, 1H, J= 6.0, 6.8 Hz), 2.36 (dd, 1H, J= 6.0, 16.4 Hz), 2.01 (t, 1H, J= 2.4 Hz), 2.04 (ddt, 1H, J= 4.8, 5.6, 7.2 Hz), 2.41 (ddd, 1H, J= 2.4, 5.6, 17.2 Hz), 2.45 (ddd, 1H, J= 2.4, 5.6, 17.2 Hz), 2.93 (bs, 1H), 3.65 (m, 2H), 3.85 (q, 1H, J = 5.6 Hz), 4.27 (ddt, 1H, J= 1.2, 4.8, 6.8 Hz), 5.18 (dt, 1H, J= 1.2, 10.8 Hz), 5.21 (dt, 1H, J = 1.2, 17.6 Hz), 5.86 (ddd, 1H, J= 6.8, 10.8, 17.6 Hz).

### (22) Synthesis of 3S,4R,5R-4-(2'-tert-butyldimethylsilyloxyethyl)-3,5-di-(tert-butyldimethylsilyloxy)-oct-1-en-7-yne (Compound 23)

From Compound 22 (60.0 mg, 146 µmol), silyl ether Compound 23 (59.0 mg, 78%) was obtained according to the same procedure of Compound 19.
[α]²⁰ _{D} 0.90 (c 1.00, CHCl₃); ¹H NMR (400 MHz, CDCl₃) δ 0.03 (s, 3H), 0.04 (s, 6H), 0.05 (s, 3H), 0.07 (s, 3H), 0.90 (s, 3H), 0.89 (s, 27H), 1.55 (m, 2H), 1.84 (ddt, 1H, J= 3.6, 5.2, 6.4 Hz), 1.95 (t, 1H, J= 2.8 Hz), 2.36 (ddd, 1H, J= 2.8, 6.4, 16.8 Hz), 2.41 (ddd, 1H, J= 2.8, 6.4, 16.8 Hz), 3.57 (dt, 1H, J= 6.4, 8.4 Hz), 3.69 (dd, 1H, J= 6.4, 10.0 Hz), 4.01 (dt, 1H, J= 3.6, 6.4 Hz), 4.13 (t, 1H, J = 6.4 Hz), 5.11 (d, 1H, J= 10.4 Hz), 5.17 (d, 1H, J= 17.2 Hz), 5.82 (ddd, 1H, J= 6.4, 10.4, 17.2 Hz).

### (23) Synthesis of 3S,4R,5R-3,5-di-(tert-butyldimethylsilyloxy)-4-ethyl-oct-1-en-7-yne (Compound 24)

Compound 22 (100 mg, 243µmol) was dissolved in dichloromethane (2.0 mL), and DMAP (74.0 mg, 606 µmol) was added. The mixture was cooled to 0°C. 2-Mesitylene sulfonyl chloride (106 mg, 485 µmol) was slowly added with vigorous stirring, and then stirred for 12 hours at the same temperature. The reaction mixture was diluted with diethyl ether (100 mL) and washed with water (10 mL) and with saturated brine (10 mL), each three times. The aqueous layer was extracted with diethyl ether (10 mL) three times. The ether layers were combined with the previously obtained ether layer, dried over magnesium sulfate, filtered and concentrated. The thus obtained sulfon ester was subjected to the next step without further purification.

The sulfon ester was dissolved in diethyl ether (2.0 mL), cooled to 0°C. LAH (lithium aluminium hydride) (46.0 mg, 1.20 µmol) was slowly added. The mixture was stirred at the same temperature for 1 hour and at room temperature for further 3 hours. The reaction mixture was cooled to 0°C, and ethyl acetate (1 mL) and saturated aqueous ammonium chloride solution (1 mL) were added, followed by diluting with diethyl ether (100 mL). The mixture was filtered through CELITE. The filtrate was washed with saturated aqueous ammonium chloride solution (10 mL) and saturated brine (10 mL), each three times. The aqueous layer was extracted with diethyl ether (10 mL) three times. The ether layers were combined with the previously obtained ether layer, dried over magnesium sulfate, filtered and concentrated. The residue was purified by flash column chromatography (ethyl acetate/hexane 1:100) to give enyne Compound 24 (77.0 mg, 80%) as a colorless oil.
[α]²⁰ _{D} 1.95 (c 1.00, CHCl₃); ¹H NMR (400 MHz, CDCl₃) δ 0.03 (s, 3H), 0.05 (s, 3H), 0.07 (s, 3H), 0.09 (s, 3H), 0.89 (s, 18H), 0.94 (t, 3H, J= 7.6 Hz), 1.36 (m, 2H), 1.68 (ddt, 1H, J= 3.6, 5.2, 6.0 Hz), 1.95 (t, 1H, J= 2.8 Hz), 2.39 (ddd, 1H, J= 2.8, 6.4, 16.8 Hz), 2.42 (ddd, 1H, J= 2.8, 6.4, 16.8 Hz), 4.01 (dt, 1H, J= 3.6, 6.4 Hz), 4.14 (ddt, 1H, J= 1.2, 5.2, 7.2 Hz),5.08 (dt, 1H, J= 1.2, 10.4 Hz), 5.14 (dt, 1H, J= 1.2, 16.8 Hz), 5.86 (ddd, 1H, J= 7.2, 10.4, 16.8 Hz).

### (24) Synthesis of 3S,4R,5R-3,5-di-(tert-butyldimethylsilyloxy)-4-(2'-cyanoethyl)-oct-1-en-7-yne (Compound 25)

From Compound 22 (129 mg, 313 µmol), nitrile Compound 25 (80.0 mg, 61%) was obtained as a colorless oil according to the same procedure of Compound 20.
[α]²⁰ _{D} 1.40 (c 1.00, CHCl₃); ¹H NMR (400 MHz, CDCl₃) δ 0.05 (s, 3H), 0.08 (s, 6H), 0.10 (s, 3H), 0.90 (s, 18H), 1.73 (m, 2H), 1.96 (q, 1H, J= 6.4 Hz), 2.03 (t, 1H, J= 2.4 Hz), 2.38 (ddd, 1H, J= 2.4, 6.0, 16.8 Hz), 2.43 (ddd, 1H, J= 2.4, 4.8, 16.8 Hz), 2.50 (dt, 2H, J= 4.0, 8.4 Hz), 3.88 (dt, 1H, J= 4.8, 6.0 Hz), 4.18 (tt, 1H, J= 1.2, 6.4 Hz), 5.19 (dt, 1H, J = 1.2, 10.4 Hz), 5.25 (dt, 1H, J= 1.2, 17.2 Hz), 5.80 (ddd, 1H, J= 6.4, 10.4, 17.2 Hz) ; HREIMS C₂₃H₄₃O₂NSi₂ (M⁺) calcd. 421.2814, found 421.2812.

### (25) Synthesis of 3S,4R,5R-3,5-di-(tert-butyldimethylsilyloxy)-4-(3'-propanal)-oct-1-en-7-yne (Compound 26)

From the nitrile Compound 25 (80.0 mg, 190 µmol), aldehyde Compound 26 (71.0 mg, 89%) was obtained as a colorless oil according to the same procedure of Compound 21.
[α]²⁰ _{D} 0.37 (c 1.00, CHCl₃); ¹H NMR (400 MHz, CDCl₃) δ 0.04 (s, 3H), 0.06 (s, 3H), 0.07 (s, 3H), 0.10 (s, 3H), 0.89 (s, 18H), 1.69 (m, 2H), 1.87 (ddt, 1H, J= 5.2, 5.6, 6.0 Hz), 1.87 (ddt, 1H, J= 5.2, 5.6, 6.0 Hz), 1.99 (t, 1H, J= 2.8 Hz), 2.40 (ddd, 1H, J= 2.8, 6.0, 17.2 Hz), 2.43 (ddd, 1H, J= 2.8, 6.0, 17.2 Hz), 2.57 (ddd, 1H, J= 2.0, 2.8, 6.0 Hz), 2.59 (ddd, 1H, J= 2.0, 3.2, 6.0 Hz), 3.94 (dt, 1H, J = 4.4, 6.0 Hz), 4.17 (ddt, 1H, J= 1.6, 5.6, 6.8 Hz), 5.14 (dt, 1H, J= 1.6, 10.4 Hz), 5.25 (dt, 1H, J= 1.6, 17.2 Hz), 5.80 (ddd, 1H, J= 6.8, 10.4, 17.2 Hz).

### (26) Synthesis of 3S,4R,5R-4-(3'-hydroxypropyl)-3,5-di-(tert-butyldimethylsilyloxy)-oct-1-en-7-yne (Compound 27)

From the aldehyde Compound 26 (71.0 mg, 167 µmol), alcohol Compound 27 (70.0 mg, 98%) was obtained as a colorless oil according to the same procedure of Compound 22.
[α]²⁰ _{D} 1.18 (c 1.00, CHCl₃); ¹H NMR (400 MHz, CDCl₃) δ 0.04 (s, 3H), 0.06 (s, 3H), 0.07 (s, 3H), 0.10 (s, 3H), 0.89 (s, 18H), 1.39 (m, 2H), 1.57 (bs, 1H), 1.66 (q, 1H, J= 6.4 Hz), 1.85 (ddt, 1H, J= 4.8, 5.2, 5.6 Hz), 1.98 (t, 1H, J= 2.8 Hz), 2.39 (ddd, 1H, J= 2.8, 6.4, 16.4 Hz), 2.43 (ddd, 1H, J= 2.8, 6.4, 16.4 Hz), 3.61 (t, 2H, J= 6.4 Hz), 3.97 (dt, 1H, J= 4.8, 6.4 Hz), 4.16 (ddt, 1H, J = 1.2, 5.6, 7.2 Hz), 5.12 (dt, 1H, J= 1.2, 9.6 Hz), 5.17 (dt, 1H, J= 1.6, 16.8 Hz), 5.84 (ddd, 1H, J= 7.2, 9.6, 16.8 Hz); HREIMS C₂₃H₄₆O₃Si₂ (M⁺) calcd. 426.2985, found 426.2977.

### (27) Synthesis of 3S,4R,5R-4-(3'-tert-butyldimethylsilyloxypropyl)-3,5-di-(tert-butyldimethylsilyloxy)-oct-1-en-7-yne (Compound 28)

From the alcohol Compound 27 (60.0 mg, 141 µmol), silyl ether Compound 28 (63.0 mg, 83%) was obtained as a colorless oil according to the same procedure of Compound 23.
[α]²⁰ _{D} 1.00 (c 1.00, CHCl₃) ; ¹H NMR (400 MHz, CDCl₃) δ 0.03 (s, 3H), 0.04 (s, 6H), 0.05 (s, 3H), 0.07 (s, 3H), 0.09 (s, 3H), 0.89 (s, 27H), 1.32 (m, 2H), 1.56 (m, 2H), 1.75 (ddt, 1H, J= 4.0, 6.4, 6.8 Hz), 1.95 (t, 1H, J= 2.8 Hz), 2.38 (ddd, 1H, J= 2.8, 6.4, 16.8 Hz), 2.42 (ddd, 1H, J= 2.8, 6.4, 16.8 Hz), 3.56 (t, 2H, J= 6.8 Hz), 4.03 (dt, 1H, J= 3.6, 6.0 Hz), 4.12 (dd, 1H, J = 6.4, 7.6 Hz), 5.08 (d, 1H, J= 10.0 Hz), 5.14 (d, 1H, J= 17.2 Hz), 5.84 (ddd, 1H, J= 7.6, 10.0, 17.2 Hz).

### (28) Synthesis of 3S,4R,5R-3,5-di-(tert-butyldimethylsilyloxy)-4-propyl-oct-1-en-7-yne (Compound 29)

From the alcohol Compound 27 (70.0 mg, 164 µmol), enyne Compound 29 (54.0 mg, 80%) was obtained as a colorless oil according to the same procedure of Compound 24.
[α]²⁰ _{D} 2.24 (c 1.00, CHCl₃); ¹H NMR (400 MHz, CDCl₃) δ 0.02 (s, 3H), 0.05 (s, 6H), 0.06 (s, 3H), 0.09 (s, 3H), 0.87 (t, 3H, J= 7.2 Hz), 0.89 (s, 18H), 1.27 (m, 2H), 1.36 (m, 2H), 1.76 (ddt, 1H, J= 4.0, 5.2, 6.0 Hz), 1.95 (t, 1H, J= 2.8 Hz), 2.39 (ddd, 1H, J= 2.8, 6.4, 17.6 Hz), 2.41 (ddd, 1H, J= 2.8, 6.4, 17.6 Hz), 3.99 (dt, 1H, J= 4.0, 6.4 Hz), 4.12 (ddt, 1H, J= 1.2, 6.0, 8.0 Hz), 5.07 (dt, 1H, J= 1.2, 10.4 Hz), 5.13 (dt, 1H, J= 1.2, 17.2 Hz), 5.85 (ddd, 1H, J= 8.0, 10.4, 17.2 Hz).

### (Example 2) Synthesis of vitamin D derivatives having a substituent at the 2α-position.

Example 2 was done by the following reaction scheme:

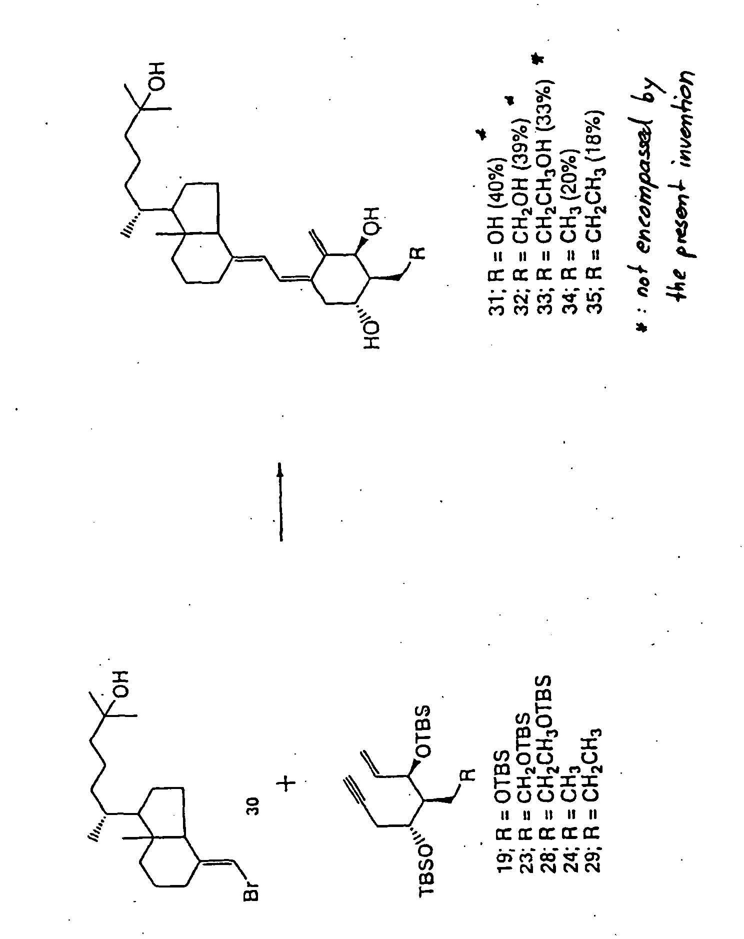

### (1) Reference Synthesis of (5Z,7E)-(lS,2S,3R,20R)-9,10-seco-5,7,10(19)-cholestatriene-2-hydroxymethyl-1,3,25-triol (Reference Compound 31)

The silyl ether Compound 19 (20.0 mg, 39.1 µmol) and a vinyl bromide 30 (20.0 mg, 58.0 µmol), which corresponds to a compound of Formula (I) in which R¹ is a 4-hydroxy-4-methylpentyl group, were dissolved in triethylamine/toluene (3:1, 2.0 mL). Tris(dibenzylideneacetone)-dipalladium(O)-chloroform addition product (4.0 mg, 3.86 µmol) and triphenylphosphine (10.0 mg, 38.1 µmol) were added. The mixture was stirred at room temperature for 15 min. and then refluxed for 2 hours. The reaction mixture was filtered through silica gel; the filtrate was concentrated and roughly purified by thin layer chromatography (ethyl acetate/hexane 1:4) to give a protected derivative as a colorless solid. The crude protected body was subjected to the next step without further purification.

The protected derivative was dissolved in methanol (2.0 mL), cooled to 0°C and (+)-10-camphorsulfonic acid (10.0 mg, 43 µmol) was added. The mixture was stirred at the same temperature for 1 hour and then returned to room temperature, followed by stirring for further 12 hours. The reaction mixture was diluted with ethyl acetate (10 mL) and washed with saturated aqueous sodium bicarbonate solution (1.0 mL) and saturated brine (1.0 mL), each three times. The aqueous layer was extracted with ethyl acetate (2 mL) three times. The ethylacetate layers were combined with the previously obtained organic layer, dried over magnesium sulfate, filtered and concentrated. The residue was purified by thin layer chromatography (ethyl acetate/hexane 1:4) to give Compound 31 (7.0 mg, 40%) as a colorless solid.
[α]²⁰ _{D} 12.95 (c 0.0085, CHCl₃); ¹H NMR (400 MHz, CDCl₃) δ 0.53 (s, 3H), 0.94 (d, 3H, J= 6.8 Hz), 2.31 (m, 2H), 2.67 (dd, 1H, J= 4.4, 12.8 Hz), 2.73 (bs, 1H), 2.84 (m, 1H), 4.01 (m, 2H), 4.24 (m, 1H), 4.47 (bs, 1H), 5.02 (d, 1H, J= 2.0 Hz), 5.30 (d, 1H, J= 2.0 Hz), 5.98 (d, 1H, J= 10.8 Hz), 6.45 (d, 1H, J= 10.8 Hz) ; HREIMS C₂₈H₄₄O₃ (M⁺-H₂O) calcd. 428.3290, found 428.3287.

### (2) Synthesis of (5Z,7E)-(1S,2S,3R,20R)-9,10-seco-5,7,10(19)-cholestatriene-2-(2'-hydroxyethyl)-1,3,25-triol (Compound 32)

From the silyl ether Compound 23 (25.0 mg, 47.5 µmol), Reference Compound 32 (8.5 mg, 39%) was obtained as a colorless solid according to the same procedure of Compound 31.
[α]²⁰_{D} 13.85 (c 0.00722, CHCl₃); ¹H NMR (400 MHz, CDCl₃) δ 0.53 (s, 3H), 0.94 (d, 3H, J= 6.4 Hz), 2.26 (dd, 1H, J= 8.0, 12.8 Hz), 2.53 (bs, 1H), 2.66 (dd, 1H, J= 4.0, 13.2 Hz), 2.83 (m, 1H), 3.79 (m, 2H), 3.94 (m, 1H), 4.37 (d, 1H, J= 2.0 Hz), 5.02 (d, 1H, J= 1.2 Hz), 5.30 (bs, 1H), 6.01 (d, 1H, J= 10.8 Hz), 6.40 (d, 1H, J= 10.8 Hz) ; HREIMS C₂₉H₄₈O₄ (M⁺) calcd. 460.3553, found 460.3557.

### (3) Synthesis of (5Z,7E)-(1S,2S,3R,20R)-9,10-seco-5,7,10(19)-cholestatriene-2-(3'-hydroxypropyl)-1,3,25-triol(Compound 33)

From the silyl ether Compound 28 (25.0 mg, 46.3 µmol), Reference Compound 33 (7.2 mg, 33%) was obtained as a colorless solid according to the same procedure of Compound 31.
[α]²⁰ _{D} 161.29 (c 0.00186, CHCl₃); ¹H NMR (400 MHz, CDCl₃) δ 0.53 (s, 3H), 0.94 (d, 3H, J= 6.4 Hz), 2.25 (dd, 1H, J= 8.4, 13.2 Hz), 2.66 (dd, 1H, J= 4.0, 13.2 Hz), 2.83 (m, 1H), 3.70 (t, 2H, J= 5.6 Hz), 3.90 (dt, 1H, J= 4.0, 8.0 Hz), 4.38 (d, 1H, J= 3.6 Hz), 5.00 (d, 1H, J= 1.6 Hz), 5.28 (d, 1H, J= 1.6 Hz), 6.00 (d, 1H, J= 11.2 Hz), 6.40 (d, 1H, J= 11.2 Hz).

### (4) Synthesis of (5Z,7E)-(1S,2S,3R,20R)-9,10-seco-5,7,10(19)-cholestatriene-2-ethyl-1,3,25-triol (Compound 34)

From the enyne Compound 24 (30.0 mg, 75.8 µmol), Compound 34 (6.8 mg, 20%) was obtained as a colorless solid according to the same procedure of Compound 31.
¹H NMR (400 MHz, CDCl₃) δ 0.53 (s, 3H), 0.94 (d, 3H, J= 6.0 Hz), 0.95 (t, 3H, J= 7.2 Hz), 2.24 (dd, 1H, J= 8.8, 12.8 Hz), 2.66 (dd, 1H, J= 4.0, 13.2 Hz), 2.83 (m, 1H), 3.89 (m, 1H), 4.37 (bs, 1H), 4.99 (d, 1H, J= 1.6 Hz), 5.27 (bs, 1H), 6.00 (d, 1H, J= 11.2 Hz), 6.40 (d, 1H, J= 11.2 Hz); HREIMS C₂₉H₄₈O₃ (M⁺) calcd. 444.3603, found 460.3604.

### (5) Synthesis of (5Z,7E)-(1S,2S,3R,20R)-9,10-seco-5,7,10(19)-cholestatriene-2-propyl-1,3,25-triol (Compound 35)

From the enyne Compound 29 (30.0 mg, 73.2 µmol), Compound 35 (6.2 mg, 18%) was obtained as a colorless solid according to the same procedure of Compound 31.
¹H NMR (400 MHz, CDCl₃) δ 0.53 (s, 3H), 0.94 (d, 3H, J= 6.4 Hz),1.01 (t, 3H, J= 6.8 Hz), 2.24 (dd, 1H, J= 8.8, 12.4 Hz), 2.66 (dd, 1H, J= 4.0, 13.2 Hz), 2.83 (m, 1H), 3.89 (m, 1H), 4.39 (bs, 1H), 4.99 (d, 1H, J= 1.6 Hz), 5.27 (bs, 1H), 6.00 (d, 1H, J= 11.2 Hz), 6.40 (d, 1H, J= 11.2 Hz). ; HREIMS C₃₀H₅₀O₃ (M⁺) calcd. 458.3760, found 458.3755.

### (Test example 1) Assay for binding to vitamin D receptor (VDR)

Bovine thymus 1α,25-dihydroxyvitamin D₃ receptor was purchased from YAMASA SHOYU KABUSHIKI KAISHA (lot.110431) and 1 ampule (approximately 25 mg) of the receptor was dissolved in 55 mL of 0.05 M phosphate 0.5M potassium buffer (pH 7.4) just before use. Ethanol solutions (50 µl) of 1α,25-dihydroxyvitamin D₃ and Compounds 31-35 of the present invention at various concentrations were prepared, mixed with aliquots (500 µl, 0.23 mg protein) of the receptor solution and pre-incubated at room temperature for 1 hour. To the resultant mixtures, [³H]-1α,25-dihydroxyvitamin D₃ was added at the final concentration of 0.1 nM, followed by incubation overnight at 4°C.

The reaction mixtures were treated with dextran coated charcoal for 30 minutes at 4°C to separate the bound and free forms of [³H]-1α,25-dihydroxyvitamin D₃, and centrifuged at 3000 rpm for ten minutes. Each of the resultant supernatants (500 µl) was mixed with ACS-II (9.5 ml) (AMERSHAM, England) for radioactivity measurement.

The binding properties of Compounds 31-35 of the present invention were expressed in relative values with that of 1α,25-dihydroxyvitamin D₃ taken as 100. The results are shown in Table 1.

**Table 1**

| Compound | Binding property |
|---|---|
| 31 (Reference) | 20 |
| 32 (Reference) | 70 |
| 33 (Reference) | 300 |
| 34 | 40 |
| 35 | 20 |

### INDUSTRIAL APPLICABILIY

Vitamin D₃ derivatives represented by Formula (I), (II) and (III) of the present invention are novel compounds and may be useful as pharmaceutical agents. The compounds of the present invention may be useful as reagents for studying metabolism of active vitamin D₃, that is, 1α,25-dihydroxyvitamin D₃.

## Claims

1. A vitamin D derivative represented by Formula (I): 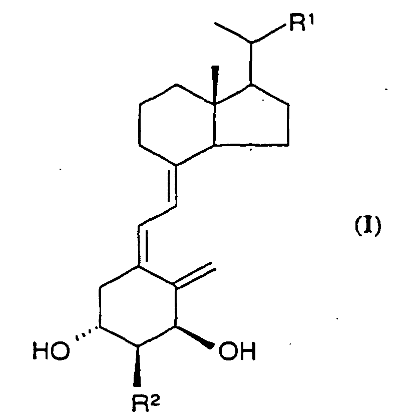 wherein R¹ represents a saturated aliphatic C₁₋₁₅hydrocarbon group optionally substituted with 1 to 3 hydroxy or protected hydroxy groups; and
R² represents a saturated aliphatic C₁₋₁₀hydrocarbon group optionally substituted with one or more substituents, which may be the same or different and which are selected from the group consisting of a cyano group, a C₁₋₆ lower alkoxy group, an amino group and an acylamino group, provided that when R² represents a saturated aliphatic C₁ hydrocarbon group, R² is substituted with at least one of said substituents.

2. The vitamin D derivative of claim 1 which is represented by Formula (II): 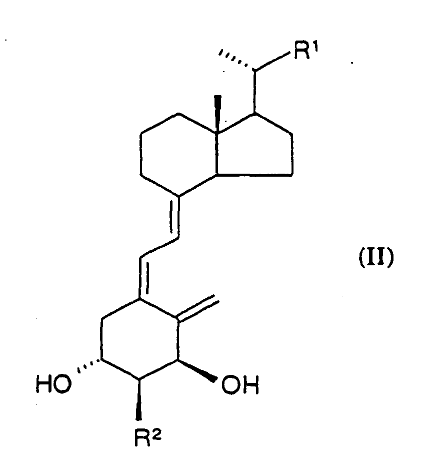 wherein R¹ and R² are as defined in claim 1.

3. The vitamin D derivative of claim 1 which is represented by Formula (III): 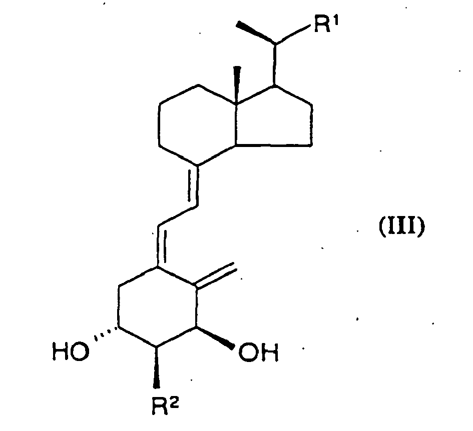 wherein R¹ and R² are as defined in claim 1.

4. The vitamin D derivative according to any one of claims 1 to 3, wherein R¹ is a 4-hydroxy-4-methylpentyl group.

5. A pharmaceutical composition comprising a vitamin D derivative according to any one of claims 1 to 4 as an active ingredient.

6. Use of a compound as defined in any one of claims 1-5 for the preparation of a pharmaceutical composition for the treatment of diseases associated with abnormal calcium metabolism, an antitumor agent or immunomodulator.

7. A composition comprising a compound as defined in any one of claims 1-5 for the treatment of diseases associated with abnormal calcium metabolism, an antitumor agent or immunomodulator.

## Patentansprüche

1. Vitamin-D-Derivat der Formel (I): 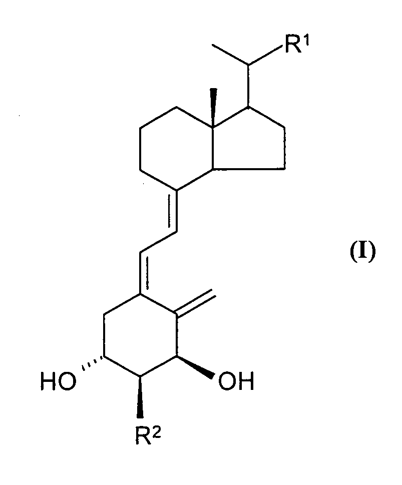 wobei R¹ einen gesättigten aliphatischen C₁₋₁₅-Kohlenwasserstoffrest darstellt, gegebenenfalls substituiert mit 1 bis 3 Hydroxy- oder geschützten Hydroxygruppen; und R² einen gesättigten aliphatischen C₁₋₁₀-Kohlenwasserstoffrest darstellt, gegebenenfalls substituiert mit einem oder mehreren Substituenten, welche gleich oder verschieden sein können und ausgewählt sind aus einer Cyanogruppe, einem C₁₋₆-Niederalkoxyrest, einer Aminogruppe und einem Acylaminorest, mit der Maßgabe, dass, wenn R² ein gesättigter aliphatischer C₁-Kohlenwasserstoffrest ist, R² mit mindestens einem dieser Substituenten substituiert ist.

2. Vitamin-D-Derivat nach Anspruch 1 der Formel (II): 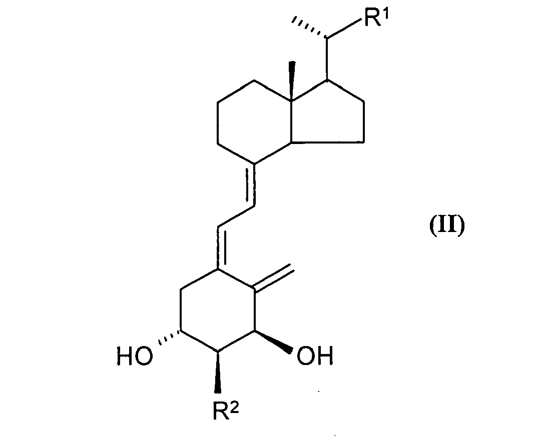 wobei R¹ und R² wie in Anspruch 1 definiert sind.

3. Vitamin-D-Derivat nach Anspruch 1 der Formel (III): 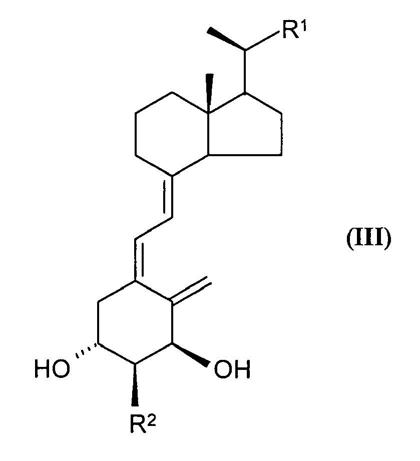 wobei R¹ und R² wie in Anspruch 1 definiert sind.

4. Vitamin-D-Derivat nach einem der Ansprüche 1 bis 3, wobei R¹ eine 4-Hydroxy-4-methylpentylgruppe ist.

5. Arzneimittel umfassend ein Vitamin-D-Derivat nach einem der Ansprüche 1 bis 4 als Wirkstoff.

6. Verwendung einer Verbindung wie in einem der Ansprüche 1 bis 5 definiert für die Herstellung eines Arzneimittels zur Behandlung von Krankheiten, welche mit abnormalem Calcium-Metabolismus, einem Antitumormittel oder einem Immunmodulator in Zusammenhang stehen.

7. Zusammensetzung umfassend eine Verbindung wie in einem der Ansprüche 1 bis 5 definiert zur Behandlung von Krankheiten, welche mit abnormalem Calcium-Metabolismus, einem Antitumormittel oder einem Immunmodulator in Zusammenhang stehen.

## Revendications

1. Dérivé de la vitamine D représenté par la formule (I) : 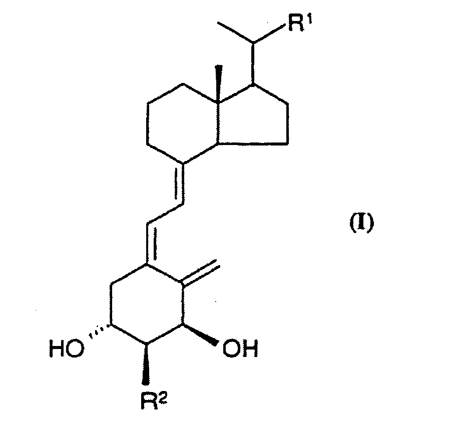 dans laquelle R¹ représente un groupe hydrocarboné aliphatique en C₁₋₁₅ saturé éventuellement substitué par 1 à 3 groupes hydroxy ou hydroxy protégé ; et R² représente un groupe hydrocarboné aliphatique en C₁₋₁₀ saturé, éventuellement substitué par un ou plusieurs substituants, qui peuvent être identiques ou différents et qui sont sélectionnés parmi le groupe constitué d'un groupe cyano, un groupe alkoxyle inférieur en C₁₋₆, un groupe amino et un groupe acylamino, à la condition que lorsque R² représente un groupe hydrocarboné aliphatique saturé en C₁, R² soit substitué par au moins un desdits substituants.

2. Dérivé de la vitamine D selon la revendication 1 qui est représenté par la formule (II) : 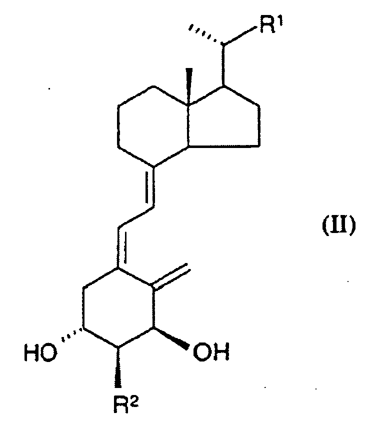 dans laquelle R¹ et R² sont tels que définis dans la revendication 1.

3. Dérivé de la vitamine D selon la revendication 1 qui est représenté par la formule (III) : 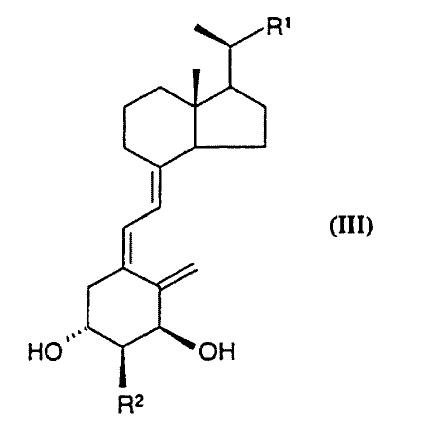 dans laquelle R¹ et R² sont tels que définis dans la revendication 1.

4. Dérivé de la vitamine D selon l'une quelconque des revendications 1 à 3, dans lequel R¹ est un groupe 4-hydroxy-4-méthylpentyle.

5. Composition pharmaceutique comprenant un dérivé de la vitamine D selon l'une quelconque des revendications 1 à 4 comme principe actif.

6. Utilisation d'un composé tel que défini dans l'une quelconque des revendications 1 à 5 pour la préparation d'une composition pharmaceutique destiné au traitement des maladies associées à un métabolisme anormal du calcium, un agent antitumoral ou un immunomodulateur.

7. Composition comprenant un composé tel que défini dans l'une quelconque des revendications 1 à 5 pour la préparation d'une composition pharmaceutique destiné au traitement des maladies associées à un métabolisme anormal du calcium, un agent antitumoral ou un immunomodulateur.
